(19)

Europäisches
Patentamt

European
Patent Office

Office européen
des brevets

(11) **EP 4 678 655 A1**

(12) **EUROPEAN PATENT APPLICATION**
published in accordance with Art. 153(4) EPC

(43) Date of publication:
**14.01.2026 Bulletin 2026/03**

(21) Application number: **24771142.7**

(22) Date of filing: **11.03.2024**

(51) International Patent Classification (IPC):
*C07K 14/47* (2006.01)    *C07K 16/18* (2006.01)
*C07K 14/725* (2006.01)    *A61K 47/68* (2017.01)
*A61P 37/00* (2006.01)    *A61P 25/28* (2006.01)
*G01N 33/68* (2006.01)    *A61K 39/00* (2006.01)

(52) Cooperative Patent Classification (CPC):
**A61K 39/00; A61K 47/68; A61P 25/28;
A61P 37/00; C07K 14/47; C07K 14/705;
C07K 16/18; G01N 33/68**

(86) International application number:
**PCT/KR2024/003075**

(87) International publication number:
**WO 2024/191142 (19.09.2024 Gazette 2024/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.03.2023 KR 20230031776**

(71) Applicant: **Good T Cells, Inc.
Seoul 03929 (KR)**

(72) Inventors:
• **KIM, Jung Ho
Seoul 04136 (KR)**
• **KIM, Beom Seok
Seoul 03488 (KR)**

(74) Representative: **Dragotti & Associati S.P.A.
Via Nino Bixio, 7
20129 Milano (IT)**

Remarks:
The complete document including Reference
Table(s) and the Sequence Listing(s) can be
downloaded from the EPO website

(54) **EPITOPE OF REGULATORY T CELL SURFACE ANTIGEN, AND ANTIBODY SPECIFICALLY
BINDING THERETO**

(57) The present invention relates to an antibody or antigen-binding fragment that specifically binds to the epitope of the Lrig-1 (leucine-rich and immunoglobulin-like domains 1) protein, which is an antigen present on the surface of regulatory T cells.

The antibody or antigen-binding fragment that specifically binds to the epitope of the Lrig-1 protein according to the present invention specifically binds to the epitope of the present invention present on regulatory T cells, thereby regulating the function of the regulatory T cells, maintaining or enhancing the activity of effector T cells, and can be very efficiently used for the prevention, improvement, or treatment of various diseases.

【FIG. 2】

5×FAD Tg     GA     GTC110-04     GTC310-01

EP 4 678 655 A1

## Description

Technical Field

The present invention relates to epitopes of the Lrig-1 (leucine-rich and immunoglobulin-like domains 1) protein, which is an antigen present on the surface of regulatory T cells, and antibodies or antigen-binding fragments specifically binding thereto.

Background Art

In degenerative brain and nervous system diseases such as stroke, dementia, and Alzheimer's disease, deterioration in memory, attention, cognitive ability, emotion regulation, and the like is observed, which results from death of neuronal cells and atrophy of nerve branches. Nerve branch elongation in neuronal cells leads to increased neuroplasticity, and thus plays an important role in memory and learning functions of neural circuitry. Therefore, it is predicted that active ingredients, which promote nerve branch elongation and nerve regeneration in neuronal cells, have the potential to be developed as new therapeutic agents for degenerative brain and nervous system diseases.

As the aging population rapidly increases, the incidence of degenerative brain and nervous system diseases is also on the rise. Despite innovative advances in medicine, prophylactic and therapeutic methods for degenerative brain and nervous system diseases are not yet clearly established, and no drugs have been found which have a decisive effect. Currently, therapeutic agents and treatment methods for degenerative brain and nervous system diseases are being developed; however, they often exhibit side effects and toxicity due to long-term use, and only have an effect of alleviating symptoms rather than treating the disease. Therefore, there is an urgent need to develop a material capable of achieving treatment while having decreased side effects and toxicity.

Technical Problem

An object of the present invention is to provide an epitope of the Lrig-1 (leucine-rich and immunoglobulin-like domains 1) protein present on the surface of regulatory T cells (Treg cells).

Another object of the present invention is to provide an antibody or antigen-binding fragment that can specifically bind to the aforementioned epitope.

Another object of the present invention is to provide a nucleic acid molecule encoding the epitope of the present invention; an expression vector into which the nucleic acid molecule is inserted; and a host cell line infected with the expression vector.

Another object of the present invention is to provide pharmaceutical compositions for the prevention or treatment of various diseases, comprising as an active ingredient an antibody or antigen-binding fragment capable of specifically binding to the epitope.

Another object of the present invention is to provide an antibody-drug conjugate (ADC) comprising the antibody according to the present invention and a drug for the prevention or treatment of various diseases.

Another object of the present invention is to provide a pharmaceutical composition for the prevention or treatment of brain and nervous system diseases containing a chimeric antigen receptor (CAR) as an active ingredient according to the present invention.

Another object of the present invention is to provide a diagnostic composition and diagnostic kit for brain and nervous system diseases containing the conjugated molecule according to the present invention, and a method for providing information for diagnosing brain and nervous system diseases.

Another object of the present invention is to provide pharmaceutical compositions for the prevention or treatment of various diseases, such as immune-related diseases, neurodegenerative diseases, or neuroinflammatory diseases, comprising antibody-drug conjugates as an active ingredient.

Another object of the present invention is to provide methods for preventing or treating various diseases using antibodies or antigen-binding fragments capable of specifically binding to the epitope, and antibody-drug conjugates.

However, the technical problem to be achieved by the present invention is not limited to the above-mentioned problems, and other problems that are not mentioned will be clearly understood by a skilled person in the art from the following description.

Solution to Problem

Various embodiments of the present invention are described with reference to the accompanying drawings. In the following description, various specific details, such as specific forms, compositions, and processes, are described for a complete understanding of the present invention. However, specific embodiments may be implemented without one or more of these specific details or in combination with other known methods and forms. In other examples, known processes and manufacturing techniques are not described in detail to avoid making the present invention unnecessary or ambiguous. A reference throughout this specification to a single embodiment means that the special features, forms, compositions, or characteristics described in connection with that embodiment are included in one or more embodiments of the present invention. Therefore, the situations of embodiments described at various locations throughout this specification do not necessarily represent the same embodiments of the invention. Additionally, special features, forms, compositions, or characteristics may be combined in any suitable manner in one or more embodiments.

The present invention relates to an epitope of the Lrig-1 (leucine-rich and immunoglobulin-like domains 1) protein or an antibody or antigen-binding fragment specifically binding to the epitope.

In the present invention, the "epitope" refers to a sequence of amino acids in an antigen wherein the amino acids (or a subset thereof) in the sequence are specifically recognized by an antibody or binding fragment, for example an antibody or binding fragment described herein. An epitope may comprise one or more antigenic determinants. For example, an antibody generated against an isolated peptide corresponding to a conformational epitope recognizes part or all of said epitope sequence.

In the present invention, the "Lrig-1 protein" is a transmembrane protein consisting of 1091 amino acids present on the surface of regulatory T cells, and is composed of leucine-rich repeats (LRRs) and three immunoglobulin-like domains on the extracellular or lumen side, a cell transmembrane sequence, and a cytoplasmic tail portion. The LRIG gene family includes LRIG1, LRIG2, and LRIG3, and the amino acids therebetween are highly conserved. The LRIG1 gene is highly expressed in normal skin and can be expressed in basal and hair follicle cells to regulate proliferation of epithelial stem cells. Therefore, the LRIG1 gene plays an important role in maintaining homeostasis of the epidermis, and its absence may develop psoriasis or skin cancer. It has been reported that in a case where chromosome 3p14.3 portion in which LRIG1 is located is cut off, there is a possibility of developing into cancer cells. In fact, it was identified that expression of LRIG1 is greatly decreased in renal cell carcinoma and cutaneous squamous cell carcinoma. Recently, it has been also found that Lrig-1 is expressed in only about 20 to 30% of cancers. On the other hand, for the purpose of the present invention, the Lrig-1 protein may be, but is not limited to, a protein present in humans or mice.

As an example of the present invention, the Lrig-1 protein may be a Lrig-1 protein derived from mammals, including primates such as humans and monkeys, rodents such as mice and rats, or the like.

As an example of the present invention, the Lrig-1 protein may be a human-derived Lrig-1 protein represented by SEQ ID NO: 1, which may be encoded by the nucleic acid sequence represented by SEQ ID NO: 2, without being limited thereto.

As another example of the present invention, the Lrig-1 protein may be a mouse-derived Lrig-1 protein represented by SEQ ID NO: 3, which may be encoded by the nucleic acid sequence represented by SEQ ID NO: 4, without being limited thereto.

As another example of the present invention, the Lrig-1 protein may be an extracellular domain of the Lrig-1 protein, without being limited thereto.

The Lrig-1 extracellular domain in the present invention may be an extracellular domain of Lrig-1 protein derived from mammals including primates such as humans and monkeys, rodents such as mice and rats, or the like. For the purpose of the present invention, the extracellular protein of the Lrig-1 protein may be an extracellular domain of Lrig-1 protein derived from a human or mouse, without being limited thereto.

As an example of the present invention, the extracellular domain of the Lrig-1 protein may be represented by SEQ ID NO: 5, which corresponds to amino acid positions 35 to 794 in the amino acid sequence of the human-derived Lrig-1 protein, without being limited thereto.

As another example of the present invention, the extracellular domain of the Lrig-1 protein may be represented by SEQ ID NO: 6, which corresponds to amino acid positions 35 to 794 in the amino acid sequence of the mouse-derived Lrig-1 protein, without being limited thereto.

Hereinafter, the present invention will be described in more detail.

In accordance with one embodiment of the present invention, there is provided an epitope of the Lrig-1 protein, the epitope comprising at least one polypeptide selected from the group consisting of polypeptides consisting of the amino acid sequence represented by SEQ ID NOs: 24, 27, 30, 31, 34, 38, 42 and 51.

As an example of the present invention, the epitope of the Lirg-1 protein may be an epitope comprising at least one polypeptide selected from the group consisting of polypeptides consisting of the amino acid sequence represented by SEQ ID NOs: 23 to 60, without being limited thereto.

As another example of the present invention, the epitope of the Lirg-1 protein may be an epitope comprising at least one polypeptide selected from the group consisting of polypeptides consisting of the amino acid sequence represented by SEQ ID NOs: 41 to 45, without being limited thereto.

The epitope of the present invention may be a conformational epitope.

The "conformational epitope" of the present invention consists of a discontinuous amino acid sequence, unlike a one-dimensional linear epitope consisting of a continuous sequence. This conformational epitope reacts with the three-dimensional structure of the antigen-binding site of an antibody.

Nucleic acid molecules of the present invention include all nucleic acid molecules having polynucleotide sequences which are translated into the amino acid sequences of the polypeptides provided in the present invention, as known to those skilled in the art. Therefore, various polynucleotide sequences with open reading frames (ORFs) may be produced, which are also all included in the nucleic acid molecules of the present invention.

Another embodiment of the present invention provides an expression vector into which the isolated nucleic acid molecule provided in the present invention has been inserted.

In the present invention, the "vector" is any nucleic acid molecule capable of transporting another nucleic acid linked thereto. One type of vector is a "plasmid," which refers to a circular double-stranded DNA to which an additional DNA segment may be ligated. Another type of vector is a phage vector. Yet another type of vector is a viral vector, where an additional DNA segment can be ligated into the viral genome. Certain vectors are capable of autonomous replication in a host cell into which they have been introduced (for example, bacterial vectors having a bacterial origin of replication are episomal mammalian vectors). Other vectors (e.g., non-episomal mammalian vectors) can be integrated into the genome of a host cell upon introduction into the host cell, and thus are replicated along with the host genome. In addition, certain vectors are capable of directing expression of genes to which they are operatively linked. Such vectors are referred to herein as "recombinant expression vectors" or simply "expression vectors." In general, expression vectors useful in recombinant DNA techniques are often in the form of plasmids. In the present specification, the terms "plasmid" and "vector" may be used interchangeably as the plasmid is the most commonly used form of vector.

Specific examples of the expression vector in the present invention may be selected from the group consisting of commercially widely used pCDNA vectors, F, R1, RP1, Col, pBR322, ToL, Ti vectors; cosmids; phages such as lambda, lambdoid, M13, Mu, p1 P22, $Q\mu\mu$, T-even, T2, T3, T7; and plant viruses, without being limited thereto. Any expression vector known as expression vectors to those skilled in the art may be used in the present invention, and the expression vector is selected depending on the nature of the target host cell. Introduction of a vector into a host cell may be performed by calcium phosphate transfection, viral infection, DEAE-dextran-mediated transfection, lipofectamine transfection, or electroporation, without being limited thereto, and those skilled in the art may adopt and use an introduction method appropriate for the expression vector and host cell that are used. The vector may preferably contain at least one selection marker, without being limited thereto, and selection can be made using a vector that contains no selection marker, depending on whether or not a product is produced. The selection marker is chosen depending on the target host cell, which is done using methods already known to those skilled in the art, and thus the present invention has no limitation thereon.

In order to facilitate purification of the protein encoded by the nucleic acid molecule of the present invention, a tag sequence may be inserted into and fused to the expression vector. Examples of the tag include, but are not limited to, hexa-histidine tag, hemagglutinin tag, myc tag, or flag tag, and any tag known to those skilled in the art which facilitates purification may be used in the present invention.

Another embodiment of the present invention provides a host cell line transformed with the expression vector provided in the present invention.

In the present invention, the term "host cell" includes individual cells or cell cultures which may be or have been recipients of the vector(s) for incorporation of a polypeptide insert. The host cells include progeny of a single host cell, and the progeny may not necessarily be completely identical (in morphology or in genomic DNA complement) to the original parent cell due to natural, accidental, or intentional mutation. The host cells include cells transfected in vivo with the polynucleotide(s) of the present invention.

In the present invention, the host cells may include cells of mammalian, plant, insect, fungal, or cellular origin. For example, the host cells may be bacterial cells such as E. coli, Streptomyces, Salmonella typhimurium; fungal cells such as yeast cells and Pichia pastoris; insect cells such as Drosophila and Spodoptera Sf9 cells; animal cells such as Chinese hamster ovary (CHO) cells, SP2/0 (mouse myeloma), human lymphoblastoid, COS, NSO (mouse myeloma), 293T, Bowes melanoma cells, HT-1080, baby hamster kidney (BHK) cells, human embryonic kidney (HEK) cells, or PERC.6 (human retinal cells); or plant cells, without being limited thereto. In addition, any cell type known to those skilled in the art which may be used as a host cell line may be used.

The transformation method of the present invention is any method of introducing a desired vector into the host cell, and may include any known method capable of introducing the vector into the host cell. For example, a method using $CaCl_2$, electroporation, microinjection, calcium phosphate precipitation, electroporation, liposome-mediated transfection, DEAE-dextran treatment, gene bombardment, and transfection using virus, may be used, without being limited thereto.

Another embodiment of the present invention provides an antibody-drug conjugate (ADC) comprising: the binding molecule, antibody or antigen-binding fragment provided in the present invention; and a drug.

In the present invention, the "antibody-drug conjugate (ADC)" refers to a form in which the drug and the antibody are chemically linked to each other without degrading biological activity of the antibody and the drug. In the present invention, the antibody-drug conjugate denotes a form in which the drug is bound to an amino acid residue at the N-terminus of the heavy and/or light chain of the antibody, specifically, a form in which the drug is bound to an $\alpha$-amine group

at the N-terminus of the heavy and/or light chain of the antibody.

In the present invention, the "drug" may mean any substance having a certain biological activity for a cell, which is a concept including DNA, RNA, or a peptide. The drug may be in a form which contains a reactive group capable of reacting and crosslinking with an $\alpha$-amine group, and also includes a form which contains a reactive group capable of reacting and crosslinking with an $\alpha$-amine group and to which a linker is linked.

In the present invention, examples of the reactive group capable of reacting and crosslinking with the $\alpha$-amine group are not limited to particular types, as long as the reactive group can react and crosslink with an $\alpha$-amine group at the N-terminus of a heavy or light chain of an antibody. Examples of the reactive group include all types of groups known in the art, which are capable of reacting with an amine group. The reactive group may, for example, be any one of isothiocyanate, isocyanate, acyl azide, NHS ester, sulfonyl chloride, aldehyde, glyoxal, epoxide, oxirane, carbonate, aryl halide, imidoester, carbodiimide, anhydride, and fluorophenyl ester, without being limited thereto.

According to an embodiment of the present invention, there is provided a binding molecule that specifically binds to leucine-rich and immunoglobulin-like domains 1 (Lrig-1) protein present on the surface of regulatory T cells (Treg cells).

As used herein, the term "binding molecule" refers to a variable domain comprising an intact immunoglobulin that includes a monoclonal antibody, such as a chimeric, humanized, or human monoclonal antibody, or an immunoglobulin that binds to an antigen, for example, an immunoglobulin fragment that competes with intact immunoglobulins for binding to monomeric HA or trimeric HA of influenza A virus. Regardless of the structure, an antigen-binding fragment binds to the same antigen recognized by intact immunoglobulins. The antigen-binding fragment may include a peptide or polypeptide which contains, out of the amino acid sequence of the binding molecule, an amino acid sequence of two or more contiguous residues, 20 or more contiguous amino acid residues, 25 or more contiguous amino acid residues, 30 or more contiguous amino acid residues, 35 or more contiguous amino acid residues, 40 or more contiguous amino acid residues, 50 or more contiguous amino acid residues, 60 or more contiguous amino acid residues, 70 or more contiguous amino acid residues, 80 or more contiguous amino acid residues, 90 or more contiguous amino acid residues, 100 or more contiguous amino acid residues, 125 or more contiguous amino acid residues, 150 or more contiguous amino acid residues, 175 or more contiguous amino acid residues, 200 or more contiguous amino acid residues, or 250 or more contiguous amino acid residues. The term "antigen-binding fragment", in particular, includes Fab, F(ab'), F(ab')2, Fv, dAb, Fd, complementarity determining region (CDR) fragments, single-chain antibodies (scFvs), bivalent single-chain antibodies, single-chain phage antibodies, unibodies, diabodies, triabodies, tetrabodies, polypeptides containing one or more fragments of immunoglobulin which is sufficient for a particular antigen to bind to the polypeptide, and the like. The fragment may be produced synthetically or by enzymatic or chemical digestion of a complete immunoglobulin, or may be produced by genetic engineering methods using recombinant DNA techniques. Production methods are well known in the art.

In the present invention, the binding molecule may be a binding molecule, comprising:

a heavy chain variable region that contains a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7; and

a light chain variable region that contains a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

In the present invention, the binding molecule may be a binding molecule, comprising a heavy chain variable region consisting of the amino acid sequence represented by SEQ ID NO: 11; and
a light chain variable region consisting of the amino acid sequence represented by SEQ ID NO: 12.

In the present invention, the binding molecule may be a binding molecule, comprising:

a heavy chain variable region that contains a heavy chain CDR1 encoded by the nucleotide sequence represented by SEQ ID NO: 13, a heavy chain CDR2 encoded by the nucleotide sequence represented by SEQ ID NO: 14, and a heavy chain CDR3 encoded by the nucleotide sequence represented by SEQ ID NO: 15; and
a light chain variable region that contains a light chain CDR1 encoded by the nucleotide sequence represented by SEQ ID NO: 16, a light chain CDR2 encoded by the nucleotide sequence represented by SEQ ID NO: 17, and a light chain CDR3 encoded by the nucleotide sequence represented by SEQ ID NO: 18.

In the present invention, the binding molecule may be binding molecule, comprising:

a heavy chain variable region encoded by the nucleotide sequence represented by SEQ ID NO: 19; and

a light chain variable region encoded by the nucleotide sequence represented by SEQ ID NO: 20.

In the present invention, the binding molecule may further comprise a fragment crystallization (Fc) region or a constant region. Here, the Fc region may be an Fc region of an IgA, IgD, IgE, IgM, IgG1, IgG2, IgG3, or IgG4 antibody, or may be derived therefrom. Alternatively, the Fc region may be a hybrid Fc region.

In the present invention, the Fc region may be an Fc region of a mammalian-derived IgA, IgD, IgE, IgM, IgG1, IgG2, IgG3, or IgG4 antibody, and may preferably be an Fc region of a human-derived IgA, IgD, IgE, IgM, IgG1, IgG2, IgG3, or IgG4 antibody. However, the Fc region is not limited thereto.

As an example of the present invention, the constant region may be a mouse-derived IgG2a constant region represented by SEQ ID NO: 21, but is not limited thereto.

As an example of the present invention, the constant region may be a mouse-derived immunoglobulin kappa constant region represented by SEQ ID NO: 22, but is not limited thereto.

As an example of the present invention, the constant region may be a human-derived IgG1 constant region represented by SEQ ID NO: 23 or 24, but is not limited thereto.

As an example of the present invention, the constant region may be a human-derived immunoglobulin kappa constant region represented by SEQ ID NO: 25 or 26, but is not limited thereto.

As an example of the present invention, the constant region may be a human-derived IgG2 constant region represented by SEQ ID NO: 27, but is not limited thereto.

As an example of the present invention, the constant region may be a human-derived IgG3 constant region represented by SEQ ID NO: 28, but is not limited thereto.

As an example of the present invention, the constant region may be a human-derived IgG4 constant region represented by SEQ ID NO: 29, but is not limited thereto.

As an example of the present invention, the constant region may be a constant region represented by SEQ ID NO: 30, but is not limited thereto.

As an example of the present invention, the constant region may be a constant region represented by SEQ ID NO: 31, but is not limited thereto.

As an example of the present invention, the Fc region may be a human-derived immunoglobulin lambda constant region, but is not limited thereto.

In the present invention, the "hybrid Fc" may be derived from a combination of human IgG subclasses or a combination of human IgD and IgG. In a case where the hybrid Fc binds to a biologically active molecule, polypeptide, or the like, the hybrid Fc has effects of not only increasing a serum half-life of the biologically active molecule, but also increasing an expression level of the polypeptide when a nucleotide sequence encoding the Fc-polypeptide fusion protein is expressed.

As an example of the present invention, the hybrid Fc region may be hybrid Fc represented by SEQ ID NO: 32, but is not limited thereto.

In the binding molecule of the present invention, the Fc or constant region may be linked, via a linker, to the variable region. Here, the linker may be linked to the C-terminus of the Fc or constant region, and the N-terminus of the binding molecule of the present invention may be linked to the linker. However, the present invention is not limited thereto.

In the present invention, the "linker" may contain a sequence that can be cleaved by an enzyme that is overexpressed in a tissue or cell having a target disease. In a case where the linker may be cleaved by the overexpressed enzyme as described above, it is possible to effectively prevent activity of a polypeptide from decreasing due to the Fc or constant region. In the present invention, an example of the linker may be preferably a peptide linker consisting of 33 amino acids located in the 282nd to 314th portion of human albumin which is most abundantly present in the blood, and more preferably a peptide linker consisting of 13 amino acids located in the 292nd to 304th portion of the human albumin. Such portions are portions which are mostly exposed to the outside in three-dimensional structure, and thus have a minimum possibility of inducing an immune response in the body. However, the linker is not limited thereto.

The binding molecule of the present invention may further comprise a heavy chain constant region consisting of an amino acid sequence selected from the group consisting of SEQ ID NOs: 21, 23, 24, 27, 28, 29, 30, and 32.

The binding molecule of the present invention may further comprise a light chain constant region consisting of the amino acid sequence represented by SEQ ID NO: 22, 25, 26, and 31.

The binding molecule of the present invention may further comprise:

a heavy chain constant region consisting of the amino acid sequence represented by SEQ ID NO: 21; and

a light chain constant region consisting of the amino acid sequence represented by SEQ ID NO: 22.

The binding molecule of the present invention may further comprise:

a heavy chain constant region represented by an amino acid sequence represented by SEQ ID NO: 23, 24, 27, 28, 29, and 30; and

a light chain constant region represented by an amino acid sequence represented by SEQ ID NO: 25, 26, and 31.

The binding molecule of the present invention may further comprise:

a heavy chain constant region consisting of the amino acid sequence represented by SEQ ID NO: 24; and
a light chain constant region consisting of the amino acid sequence represented by SEQ ID NO: 26.

The binding molecule of the present invention may further comprise:

a heavy chain constant region consisting of the amino acid sequence represented by SEQ ID NO: 30; and

a light chain constant region consisting of the amino acid sequence represented by SEQ ID NO: 31.

The binding molecule of the present invention may further comprise:
a heavy chain constant region consisting of the amino acid sequence represented by SEQ ID NO: 32.
The binding molecule of the present invention may further comprise:

a heavy chain constant region encoded by the nucleotide sequence represented by SEQ ID NO: 33 or 35; and

a light chain constant region encoded by the nucleotide sequence represented by SEQ ID NO: 34 or 36.

The binding molecule of the present invention may be a binding molecule comprising a heavy chain consisting of the amino acid sequence represented by SEQ ID NO: 37 or 38 and a light chain consisting of the amino acid sequence represented by SEQ ID NO: 39 or 40.
The binding molecule of the present invention may be a binding molecule comprising a heavy chain consisting of the amino acid sequence represented by SEQ ID NO: 37 and a light chain consisting of the amino acid sequence represented by SEQ ID NO: 39.
The binding molecule of the present invention may be a binding molecule comprising a heavy chain consisting of the amino acid sequence represented by SEQ ID NO: 38 and a light chain consisting of the amino acid sequence represented by SEQ ID NO: 40.
The binding molecule of the present invention may be a binding molecule comprising a heavy chain encoded by the nucleotide sequence represented by SEQ ID NO: 41 or 43 and a light chain encoded by the nucleotide sequence represented by SEQ ID NO: 42 or 44.
The binding molecule of the present invention may be a binding molecule comprising a heavy chain encoded by the nucleotide sequence represented by SEQ ID NO: 41 and a light chain encoded by the nucleotide sequence represented by SEQ ID NO: 42.
The binding molecule of the present invention may be a binding molecule comprising a heavy chain encoded by the nucleotide sequence represented by SEQ ID NO: 43 and a light chain encoded by the nucleotide sequence represented by SEQ ID NO: 44.
The binding molecule of the present invention is characterized by being an antibody or a fragment thereof, but is not limited thereto. The antibody includes all of a monoclonal antibody, a full-length antibody, or an antibody fragment which is a portion of an antibody, has the ability to bind to Lrig-1 protein, and can compete with the binding molecule of the present invention in binding to an epitope on Lrig-1.
As used herein, the term "antibody" refers to a protein molecule which serves as a receptor that specifically recognizes an antigen, including an immunoglobulin molecule that is immunologically reactive with a particular antigen. For the purpose of the present invention, the antigen may be Lrig-1 protein present on the surface of regulatory T cells. Preferably, the antibody may specifically recognize the leucine-rich region or immunoglobulin-like domain of the Lrig-1 protein, but is not limited thereto.
In the present invention, the "immunoglobulin" has a heavy chain and a light chain, and each of the heavy chain and the light chain comprises a constant region and a variable region. The variable region of each of the light chain and the heavy chain contains three hypervariable regions called complementarity determining regions (hereinafter referred to as "CDRs") and four framework regions. The CDRs primarily serve to bind to an epitope on an antigen. The CDRs of each chain are typically referred to as CDR1, CDR2, and CDR3 sequentially starting from the N-terminus, and are also distinguished by the chain where particular CDRs are located.
In addition, as used herein, the term "monoclonal antibody" refers to an antibody molecule of a single molecular composition which is obtained from substantially the same antibody population, and exhibits single binding specificity and affinity for a particular epitope.
In the present invention, the "full-length antibody" has a structure with two full-length light chains and two full-length heavy chains in which each light chain is linked to a heavy chain by a disulfide bond, and includes IgA, IgD, IgE, IgM, and IgG. The IgG includes, as subtypes thereof, IgG1, IgG2, IgG3, and IgG4.

In addition, as used herein, the term "antigen fragment" refers to a fragment that retains an antigen-binding function, and includes Fab, Fab', F(ab')$_2$, Fv, and the like. The Fab has a structure with variable regions of light and heavy chains, a constant region of the light chain, and a first constant region (CH1 domain) of the heavy chain, and has one antigen-binding site. In addition, Fab' is different from Fab in that Fab' has a hinge region containing at least one cysteine residue at the C-terminus of the heavy chain CH1 domain. F(ab')$_2$ antibodies are produced with cysteine residues at the hinge region of Fab' forming a disulfide bond. Fv (variable fragment) refers to the smallest antibody fragment having only a heavy chain variable region and a light chain variable region. Double-chain Fv (dsFv) is configured to be such that a heavy chain variable region and a light chain variable region are linked to each other by a disulfide bond, and single-chain Fv (scFv) is configured to be such that a heavy chain variable region and a light chain variable region are covalently linked to each other, in general, via a peptide linker. The antibody fragment may be obtained as Fab or F(ab')$_2$ fragment in a case where a proteolytic enzyme, for example, papain or pepsin is used, and may be produced through a genetic recombinant technique.

In addition, in the present invention, the antibody may be, but is not limited to, a chimeric antibody, a humanized antibody, a bivalent, bispecific molecule, a minibody, a domain antibody, a bispecific antibody, an antibody mimetic, a unibody, a diabody, a triabody, or a tetrabody, or a fragment thereof. In the present invention, the "chimeric antibody" is an antibody which is obtained by recombination of a variable region of a mouse antibody and a constant region of a human antibody, and has a greatly improved immune response as compared with the mouse antibody.

In addition, as used herein, the term "humanized antibody" refers to an antibody obtained by modifying a protein sequence of an antibody derived from a non-human species so that the protein sequence is similar to an antibody variant naturally produced in humans. For example, the humanized antibody may be prepared as follows. Mouse-derived CDRs may be recombined with a human antibody-derived FR to prepare a humanized variable region, and the humanized variable region may be recombined with a constant region of a preferred human antibody to prepare a humanized antibody.

In the present invention, the binding molecule may be provided as a bispecific antibody or a bispecific antigen-binding fragment which is capable of binding to Lrig-1 protein and also binding to another protein.

In the present invention, the bispecific antibody and the bispecific antigen-binding fragment may comprise the binding molecule according to the present invention. As an example of the present invention, the bispecific antibody and the bispecific antigen-binding fragment comprise an antigen-binding domain capable of binding to Lrig-1 protein, wherein the antigen-binding domain capable of binding to Lrig-1 protein may comprise or consist of the binding molecule according to the present invention.

The bispecific antibody and the bispecific antigen-binding fragment provided in the present invention comprise an antigen-binding domain, which is a binding molecule capable of binding to Lrig-1 protein according to the present invention, and an antigen-binding domain capable of binding to another target protein. Here, the antigen-binding domain capable of binding another target protein may be an antigen-binding domain capable of binding to a protein other than Lrig-1 protein, for example, but not limited to, PD-1 or a cell surface receptor. However, the antigen-binding domain is not limited thereto.

The bispecific antibody and the bispecific antigen-binding fragment according to the present invention may be provided in any suitable format. For example, the bispecific antibody or the bispecific antigen-binding fragment may be a bispecific antibody conjugate (for example, IgG2, F(ab')2, or CovX-body), a bispecific IgG or IgG-like molecule (for example, IgG, scFv4-Ig, IgG-scFv, scFv-IgG, DVD-Ig, IgG-sVD, sVD-IgG, or 2 in 1-IgG, mAb2, or Tandemab common LC), an asymmetric bispecific IgG or IgG-like molecule (for example, kih IgG, kih IgG common LC, CrossMab, kih IgG-scFab, mAb-Fv, charge pair, or SEED-body), a small bispecific antibody molecule (for example, diabody (Db), dsDb, DART, scDb, tandAb, tandem scFv (taFv), tandem dAb/VHH, triple body, triple head, Fab-scFv, or F(ab')2-scFv2), a bispecific Fc and CH3 fusion protein (for example, taFv-Fc, di-diabody, scDb-CH3, scFv-Fc-scFv, HCAb-VHH, scFv-kih-Fc, or scFv-kih-CH3), or a bispecific fusion protein (for example, scFv2-albumin, scDb-albumin, taFv-toxin, DNL-Fab3, DNL-Fab4-IgG, DNL-Fab4-IgG-cytokine 2). The bispecific antibody and the bispecific antigen-binding fragment according to the invention may be designed and prepared by those skilled in the art.

A method for producing the bispecific antibody in the present invention comprises forming a reducing disulfide or non-reducing thioether bond, and chemical crosslinking of an antibody or antibody fragment. For example, N-succinimidyl-3-(-2-pyridyldithio)-propionate (SPDP) may be used, for example, for chemically crosslinking a Fab fragment through an SH-group at the hinge region, to generate a disulfide-linked bispecific F(ab)2 heterodimer.

In addition, an alternative method for producing the bispecific antibody in the present invention comprises fusing an antibody-producing hybridoma with, for example, polyethylene glycol, to produce quadroma cells capable of secreting bispecific antibodies.

The bispecific antibody and the bispecific antigen-binding fragment according to the invention may also be, for example, recombinantly produced by expression from a nucleic acid construct that encodes a polypeptide for an antigen-binding molecule.

For example, a DNA construct that contains a sequence encoding light and heavy chain variable domains for two antigen-binding domains (that is, light and heavy chain variable domains for an antigen-binding domain capable of binding to PD-1, and light and heavy chain variable domains for an antigen-binding domain capable of binding to another target protein), and a sequence encoding a suitable linker or dimerization domain between the antigen-binding domains may be prepared by molecular cloning techniques. Subsequently, a recombinant bispecific antibody may be produced by expression of the construct (for example, *in vitro*) in a suitable host cell (for example, a mammalian host cell), and then the expressed recombinant bispecific antibody may be optionally purified.

Antibodies may be produced by an affinity maturation process in which a modified antibody with improved affinity for an antigen as compared with an unmodified parent antibody is produced. An affinity matured antibody may be produced by a procedure known in the art.

In addition, the binding molecule provided in the present invention may include a variant of the amino acid sequence as long as the variant can specifically bind to Lrig-1 protein. For example, in order to improve binding affinity and/or other biological properties of an antibody, modifications may be made to an amino acid sequence of the antibody. Such modifications include, for example, deletions, insertions, and/or substitutions of amino acid sequence residues of the antibody.

Such amino acid variations are made based on relative similarity of amino acid side chain substituents such as hydrophobicity, hydrophilicity, charge, and size. According to analysis on sizes, shapes, and types of amino acid side chain substituents, it can be seen that arginine, lysine, and histidine are all positively charged residues; alanine, glycine, and serine have similar sizes; and phenylalanine, tryptophan, and tyrosine have similar shapes. Thus, based on these considerations, it can be said that arginine, lysine, and histidine; alanine, glycine, and serine; and phenylalanine, tryptophan, and tyrosine are biologically functional equivalents.

In introducing variations, the hydropathic index of amino acids may be considered. Each amino acid has been assigned hydropathic index depending on its hydrophobicity and charge: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). The hydropathic amino acid index is very important in conferring the interactive biological function on a protein. It is known that substitution with an amino acid having similar hydropathic index allows a protein to retain similar biological activity. In a case where variations are introduced with reference to the hydropathic index, substitutions are made between amino acids that exhibit a hydropathic index difference of preferably within ± 2, more preferably within ± 1, and even more preferably within ± 0.5.

Meanwhile, it is also well known that substitutions between amino acids having similar hydrophilicity values result in proteins with equivalent biological activity. As disclosed in US Pat. No. 4,554,101, respective amino acid residues have been assigned the following hydrophilicity values: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In a case where variations are introduced with reference to the hydrophilicity values, substitutions may be made between amino acids that exhibit a hydrophilicity value difference of preferably within ± 2, more preferably within ± 1, and even more preferably within ± 0.5.

Amino acid exchanges in proteins which do not entirely alter activity of a molecule are known in the art. The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Gln/Glu.

Given the above-described variations with biologically equivalent activity, it is interpreted that the binding molecule of the present invention also includes sequences that exhibit substantial identity with the sequences listed in the Sequence Listing.

As used herein, the term "substantial identity" refers to a sequence showing at least 61% homology, more preferably 70% homology, even more preferably 80% homology, and most preferably 90% homology when the sequence of the present invention is aligned with any other sequence so that they maximally correspond to each other, and the aligned sequence is analyzed by using an algorithm typically used in the art. Alignment methods for comparison of sequences are known in the art. NCBI Basic Local Alignment Search Tool (BLAST) is accessible from the National Center for Biological Information (NBCI), or the like, and may be used in conjunction with sequencing programs, such as blastp, blasm, blastx, tblastn, and tblastx, on the internet. BLAST is accessible at http://www.ncbi.nlm.nih.gov/BLAST/. Sequence homology comparison methods using this program can be identified online (http://www.ncbi.nlm.nih.gov/BLAST/blast_help.html).

In the present invention, the binding molecule, preferably the antibody, may be produced by a conventional method for producing an antibody, and may be produced by affinity maturation.

As used herein, the term "affinity maturation" refers to a process in which antibodies having increased affinity for an antigen are produced by activated B cells in the course of an immune response. For the purpose of the present invention, the affinity maturation allows antibodies or antibody fragments to be produced due to affinity maturation based on the principles of mutation and selection, in the same process that occurs in nature.

The binding molecule, preferably the antibody, provided in the present invention can effectively prevent, ameliorate, or treat brain and nervous system diseases, in particular, neurodegenerative diseases or neuroinflammatory diseases.

According to another embodiment of the present invention, there is provided a nucleic acid molecule encoding the binding molecule provided in the present invention.

The nucleic acid molecule of the present invention includes all nucleic acid molecules obtained by translating the amino acid sequences of the binding molecules provided in the present invention to polynucleotide sequences, as known to those skilled in the art. Therefore, various polynucleotide sequences may be prepared by an open reading frame (ORF), and all of these polynucleotide sequences are also included in the nucleic acid molecule of the present invention.

The nucleic acid molecule of the present invention may comprise a heavy chain encoded by the nucleotide sequence represented by SEQ ID NO: 41 or 43 and a light chain encoded by the nucleotide sequence represented by SEQ ID NO: 42 or 44.

The nucleic acid molecule of the present invention may comprise a heavy chain encoded by the nucleotide sequence represented by SEQ ID NO: 41 and a light chain encoded by the nucleotide sequence represented by SEQ ID NO: 42.

The nucleic acid molecule of the present invention may comprise a heavy chain encoded by the nucleotide sequence represented by SEQ ID NO: 43 and a light chain encoded by the nucleotide sequence represented by SEQ ID NO: 44.

According to another embodiment of the present invention, there is provided an expression vector into which the isolated nucleic acid molecule provided in the present invention is inserted.

In the present invention, the "vector" is a nucleic acid molecule capable of transporting another nucleic acid linked thereto. One type of vector is a "plasmid," which refers to circular double-stranded DNA into which an additional DNA segment can be ligated. Another type of vector is a phage vector. Yet another type of vector is a viral vector, where an additional DNA segment can be ligated into the viral genome. Certain vectors are capable of autonomous replication in a host cell into which they are introduced (for example, bacterial vectors having a bacterial origin of replication and episomal mammalian vectors). Other vectors (for example, non-episomal mammalian vectors) can be integrated into the genome of a host cell upon introduction into the host cell, and thus are replicated along with the host genome. In addition, certain vectors are capable of directing expression of genes to which they are operatively linked. Such vectors are referred to herein as "recombinant expression vectors" or simply "expression vectors." In general, expression vectors useful in recombinant DNA techniques are often in the form of plasmids. In the present specification, "plasmid" and "vector" may be used interchangeably as the plasmid is the most commonly used form of vector.

Specific examples of the expression vector in the present invention may be selected from, but are not limited to, the group consisting of commercially widely used pCDNA vectors, F, R1, RP1, Col, pBR322, ToL, Ti vectors; cosmids; phages such as lambda, lambdoid, M13, Mu, p1 P22, $Q\mu\mu$, T-even, T2, T3, T7; plant viruses. Any expression vector known, to those skilled in the art, as expression vectors can be used in the present invention, and the expression vector is selected depending on the nature of the target host cell. Introduction of a vector into a host cell may be performed by calcium phosphate transfection, viral infection, DEAE-dextran-mediated transfection, lipofectamine transfection, or electroporation. However, the present invention is not limited thereto, and those skilled in the art may adopt and use an introduction method appropriate for the expression vector and the host cell which are used. The vector may preferably contain at least one selection marker. However, the present invention is not limited thereto, and selection can be made using the vector that contains no selection marker, depending on whether or not a product is produced. The selection marker is selected depending on the target host cell, which is done using methods already known to those skilled in the art, and thus the present invention has no limitation thereon.

In order to facilitate purification of the nucleic acid molecule of the present invention, a tag sequence may be inserted into and fused to an expression vector. The tag includes, but is not limited to, hexa-histidine tag, hemagglutinin tag, myc tag, or flag tag, and any tag known to those skilled in the art which facilitates purification can be used in the present invention.

According to another embodiment of the present invention, there is provided a host cell line transfected with the expression vector provided in the present invention.

In the present invention, the "host cell" includes individual cells or cell cultures which may be or have been recipients of the vector(s) for incorporation of a polypeptide insert. The host cell includes progeny of a single host cell, and the progeny may not necessarily be completely identical (in morphology or in genomic DNA complement) to the original parent cell due to natural, accidental, or intentional mutation. The host cell includes cells transfected *in vivo* with the polynucleotide(s) herein.

In the present invention, the host cell may include cells of mammalian, plant, insect, fungal, or cellular origin, and may be, for example, bacterial cells such as E. coli, Streptomyces, Salmonella typhimurium; fungal cells such as yeast cells and Pichia pastoris; insect cells such as Drosophila and Spodoptera Sf9 cells; animal cells such as Chinese hamster ovary (CHO) cells, SP2/0 (mouse myeloma), human lymphoblastoid, COS, NSO (mouse myeloma), 293T, Bowes melanoma cells, HT-1080, baby hamster kidney (BHK) cells, human embryonic kidney (HEK) cells, or PERC.6 (human retinal cells); or plant cells. However, the host cell is not limited thereto, and any cell known to those skilled in the art which can be used as a host cell line is available.

According to another embodiment of the present invention, there is provided an antibody-drug conjugate (ADC) comprising the antibody provided in the present invention and a drug.

As used herein, the term "antibody-drug conjugate (ADC)" refers to a form in which the drug and the antibody are chemically linked to each other without degrading biological activity of the antibody and the drug. In the present invention, the antibody-drug conjugate denotes a form in which the drug is bound to an amino acid residue at the N-terminus of the heavy and/or light chain of the antibody, specifically, a form in which the drug is bound to an $\alpha$-amine group at the N-terminus of the heavy and/or light chain of the antibody.

In the present invention, examples of the reactive group capable of reacting and crosslinking with the $\alpha$-amine group are not particularly limited in terms of type as long as the reactive group can react and crosslink with an $\alpha$-amine group at the N-terminus of a heavy or light chain of an antibody. The reactive group includes all types of groups known in the art which react with an amine group. The reactive group may, for example, be any one of isothiocyanate, isocyanate, acyl azide, NHS ester, sulfonyl chloride, aldehyde, glyoxal, epoxide, oxirane, carbonate, aryl halide, imidoester, carbodiimide, anhydride, and fluorophenyl ester, but is not limited thereto.

As used herein, the term "drug" may mean any substance having a certain biological activity for a cell, which is a concept including DNA, RNA, or a peptide. The drug may be in a form which contains a reactive group capable of reacting and crosslinking with an $\alpha$-amine group, and also includes a form which contains a reactive group capable of reacting and crosslinking with an $\alpha$-amine group and to which a linker is linked.

In the present invention, the drugs may include any type of drug capable of treating brain nervous system diseases, particularly neurodegenerative diseases or neuroinflammatory diseases.

Another embodiment of the present invention provides a binding molecule that binds specifically to an epitope of the present invention.

The "binding molecule" of the present invention may be either an antibody or an antigen-binding fragment, without being limited thereto.

In the present invention, the antibody is a full-length antibody or a part of the antibody, has the ability to bind to the Lrig-1 protein, and includes any antibody fragment that binds to the Lrig-1 antigen determining region in a manner competitive with the binding molecule of the present invention.

In the present invention, the term "antibody" refers to a protein molecule which serves as a receptor that specifically recognizes an antigen, including an immunoglobulin molecule that is immunologically reactive with a particular antigen. For the purpose of the present invention, the antigen may be Lrig-1 protein present on the surfaces of regulatory T cells. Preferably, the antibody may be one that specifically recognizes the leucine-rich region or immunoglobulin-like domain of the Lrig-1 protein, without being limited thereto.

In the present invention, the "immunoglobulin" has a heavy chain and a light chain, and each of the heavy chain and the light chain comprises a constant region and a variable region. The variable region of each of the light chain and the heavy chain contains three hypervariable regions, called complementarity determining regions (hereinafter referred to as "CDRs"), and four framework regions. The CDRs primarily serve to bind to an antigenic determinant (epitope) of an antigen. The CDRs of each chain are typically referred to as CDR1, CDR2, and CDR3, numbered sequentially starting from the N-terminus, and are also typically identified by the chain in which the particular CDR is located.

In the present invention, the antibody or antigen-binding fragment thereof provided in the present invention is a chimeric antibody or fragment comprising heavy chain and light chain CDRs selected from the CDRs provided in the present invention, or conservative variants of the CDRs provided in the present invention.

For the purpose of the present invention, "variants" of an amino acid sequence comprise amino acid insertion variants, amino acid addition variants, amino acid deletion variants and/or amino acid substitution variants. Amino acid deletion variants that comprise the deletion at the N-terminal and/or C-terminal end of the protein are also called N-terminal and/or C-terminal truncation variants.

Amino acid insertion variants comprise insertions of single or two or more amino acids in a particular amino acid sequence. In the case of amino acid sequence variants having an insertion, one or more amino acid residues are inserted into a particular site in an amino acid sequence, but random insertion with appropriate screening of the resulting product is also possible.

Amino acid addition variants comprise amino- and/or carboxy-terminal fusions of one or more amino acids, such as 1, 2, 3, 5, 10, 20, 30, 50, or more amino acids.

Amino acid deletion variants are characterized by the removal of one or more amino acids from the sequence, such as by removal of 1, 2, 3, 5, 10, 20, 30, 50, or more amino acids. The deletions may be in any position of the protein.

Amino acid substitution variants are characterized by at least one residue in the sequence being removed and another residue being inserted in its place. Preference is given to modifications being in positions in the amino acid sequence which are not conserved between homologous proteins or peptides and/or to replacing amino acids with other ones having similar properties. Preferably, amino acid changes in protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids.

Preferably, the degree of similarity, preferably identity between a given amino acid sequence and an amino acid sequence which is a variant of said given amino acid sequence will be at least about 60%, 65%, 70%, 80%, 81%, 82%, 83%, 84%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with tools known in the art, preferably using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm, or by means of a similarity search method, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wisconsin, USA.

The percentage identity is calculated by determining the number of identical positions between the two sequences being compared, dividing this number by the number of positions compared and multiplying the result obtained by 100 so as to obtain the percentage identity between these two sequences.

Homologous amino acid sequences exhibit, according to the present invention, at least 40%, in particular at least 50%, at least 60%, at least 70%, at least 80%, at least 90% and preferably at least 95%, at least 98 or at least 99% identity of the amino acid residues.

The amino acid sequence variants described herein may readily be prepared by those skilled in the art, for example, by recombinant DNA manipulation. The manipulation of DNA sequences for preparing peptides or proteins having substitutions, additions, insertions or deletions, is well known. Furthermore, the peptides and amino acid variants described herein may be readily prepared with the aid of known peptide synthesis techniques, for example, by solid phase synthesis and similar methods.

In the present invention, the antibody or antigen binding fragment thereof provided in the present invention is a humanized antibody or fragment comprising heavy and light chain CDRs selected from the CDRs provided in the present invention, or conservative variants of the CDRs provided in the present invention.

In the present invention, the antibody or antigen binding fragment thereof provided in the present invention comprises a light chain and/or a heavy chain comprising a sequence provided in the present invention, or a conservative variant thereof. In one embodiment, the conservative variants have at least 80%, at least 85%, at least 90%, at least 95%, at least 96%, at least 97%, at least 98% or at least 99% homology to the reference sequence provided in the present invention. In one embodiment, the conservative variants comprise 1, 2, 3, 4, 5, 6, 7, 8, 9, 10, or more amino acid substitutions, insertions, or deletions.

In the present invention, the antibodies provided in the present invention, which bind specifically to the Lrig-1 protein comprise the sequences provided herein or conservative variants thereof. "Conservative variants," as used herein, include conservative amino acid substitutions, insertions, or deletions. A person skilled in the art will recognize that a conservative amino acid substitution is a substitution of one amino acid with another amino acid that has similar structural or chemical properties, such as a similar side chain, and a conservative amino acid substitution results in a sequence that retains the biological activity of the reference sequence. Exemplary conservative substitutions are well known in the art.

In the present invention, the "full-length antibody" has a structure having two full-length light chains and two full-length heavy chains, wherein each of the light chains is linked to the heavy chain via a disulfide bond. Examples of the full-length antibody include IgA, IgD, IgE, IgM, and IgG. Subtypes of the IgG include IgG1, IgG2, IgG3, and IgG4.

In addition, in the present invention, the "antigen-binding fragment" refers to a fragment having an antigen-binding function. Examples of the antigen-binding fragment include: (1) a Fab fragment consisting of a light chain variable region (VL), a heavy chain variable region (VH), a light chain constant region (CL), and a heavy chain constant region 1 (CH1); (2) a Fd fragment consisting of VH and CH1 domains; (3) a Fv fragment consisting of VL and VH domains of a single antibody; (4) a dAb fragment consisting of a VH domain; (5) an isolated CDR region; (6) a $F(ab')_2$ fragment which is a bivalent fragment including two linked Fab fragments; (7) a single-chain Fv molecule (scFv) in which a VH domain and a VL domain are linked together by a peptide linker that allows the two domains to associate to form an antigen-binding site; (8) a bispecific single-chain Fv dimer; and (9) a diabody which is a multivalent or multispecific fragment constructed by gene fusion. The antigen-binding fragment may be obtained as a Fab or F(ab')2 fragment when a proteolytic enzyme, for example, papain or pepsin is used, and the antigen-binding fragment may be produced through a genetic recombination technique.

In addition, in the present invention, the antibody or a fragment thereof may be, without limitation, a monoclonal antibody, a polyclonal antibody, a chimeric antibody, a humanized antibody, a human antibody, a bivalent-bispecific molecule, a minibody, a domain antibody, a bispecific antibody, an antibody mimetic, a unibody, a diabody, a triabody, a tetrabody, or a fragment thereof, without being limited thereto.

In the present invention, the "chimeric antibody" is an antibody which is obtained by recombination of a variable region of a mouse antibody and a constant region of a human antibody, and has a greatly improved immune response compared to the mouse antibody.

In addition, in the present invention, the "humanized antibody" refers to an antibody obtained by modifying the protein sequence of an antibody of non-human species origin so that the protein sequence is similar to an antibody variant naturally produced in humans. For example, the humanized antibody may be produced by recombining mouse-derived CDRs with a human antibody-derived FR to obtain a humanized variable region, and recombining the humanized variable region with a constant region of a desired human antibody.

In the present invention, the binding molecule may be provided as a bispecific antibody or bispecific antigen-binding fragment which is capable of binding to Lrig-1 protein and also binding to another protein.

In the present invention, the bispecific antibody and the bispecific antigen-binding fragment may comprise the binding molecule according to the present invention. As an example of the present invention, the bispecific antibody and the bispecific antigen-binding fragment comprise an antigen-binding domain capable of binding to Lrig-1 protein, wherein the antigen-binding domain capable of binding to Lrig-1 protein may comprise or consist of the binding molecule according to the present invention.

The bispecific antibody and bispecific antigen-binding fragment provided in the present invention comprise an antigen-binding domain, which is a binding molecule capable of binding to Lrig-1 protein according to the present invention, and an antigen-binding domain capable of binding to another target protein. Here, the antigen-binding domain capable of binding to another target protein may be an antigen-binding domain capable of binding to a protein other than Lrig-1 protein, for example, but not limited to, PD-1 or a cell surface receptor. However, the antigen-binding domain is not limited thereto.

The bispecific antibody and the bispecific antigen-binding fragment according to the present invention may be provided in any suitable format, for example, that described in the literature, which is incorporated herein by reference in its entirety. For example, the bispecific antibody or the bispecific antigen-binding fragment may be a bispecific antibody conjugate (for example, IgG2, F(ab')2, or CovX-body), a bispecific IgG or IgG-like molecule (for example, IgG, scFv4-Ig, IgG-scFv, scFv-IgG, DVD-Ig, IgG-sVD, sVD-IgG, or 2 in 1-IgG, mAb2, or Tandemab common LC), an asymmetric bispecific IgG or IgG-like molecule (for example, kih IgG, kih IgG common LC, CrossMab, kih IgG-scFab, mAb-Fv, charge pair, or SEED-body), a small bispecific antibody molecule (for example, diabody (Db), dsDb, DART, scDb, tandAb, tandem scFv (taFv), tandem dAb/VHH, triple body, triple head, Fab-scFv, or $F(ab')_2$-scFv2), a bispecific Fc and CH3 fusion protein (for example, taFv-Fc, di-diabody, scDb-CH3, scFv-Fc-scFv, HCAb-VHH, scFv-kih-Fc, or scFv-kih-CH3), or a bispecific fusion protein (for example, scFv2-albumin, scDb-albumin, taFv-toxin, DNL-Fab3, DNL-Fab4-IgG, DNL-Fab4-IgG-cytokine 2). The bispecific antibody and bispecific antigen-binding fragment according to the present invention may be designed and produced by those skilled in the art.

In the present invention, a method for producing the bispecific antibody comprises chemical crosslinking of an antibody or antibody fragment with a reducing disulfide or non-reducing thioether bond. For example, N-succinimidyl-3-(-2-pyridyldithio)-propionate (SPDP) may be used, for example, for chemically crosslinking a Fab fragment through an SH-group at the hinge region, to generate a disulfide-linked bispecific $F(ab)_2$ heterodimer.

In addition, an alternative method for producing the bispecific antibody according to the present invention comprises fusing an antibody-producing hybridoma with, for example, polyethylene glycol, to produce quadroma cells

**EP 4 678 655 A1**

capable of secreting bispecific antibodies.

The bispecific antibody and bispecific antigen-binding fragment according to the invention may also be, for example, recombinantly produced by expression from a nucleic acid construct that encodes a polypeptide for an antigen-binding molecule.

For example, a DNA construct that contains a sequence encoding light and heavy chain variable domains for two antigen-binding domains (that is, light and heavy chain variable domains for an antigen-binding domain capable of binding to PD-1, and light and heavy chain variable domains for an antigen-binding domain capable of binding to another target protein), and a sequence encoding a suitable linker or dimerization domain between the antigen-binding domains may be prepared by molecular cloning techniques. Subsequently, a recombinant bispecific antibody may be produced by expression of the construct (for example, in vitro) in a suitable host cell (for example, a mammalian host cell), and then the expressed recombinant bispecific antibody may be optionally purified.

Antibodies may be produced by an affinity maturation process in which a modified antibody with improved affinity for an antigen as compared with an unmodified parent antibody is produced. An affinity-matured antibody may be produced by a procedure known in the art.

In addition, the binding molecule provided in the present invention may include a variant of the amino acid sequence as long as the variant can specifically bind to Lrig-1 protein. For example, in order to improve binding affinity and/or other biological properties of an antibody, modifications may be made to an amino acid sequence of the antibody. Such modifications include, for example, deletions, insertions, and/or substitutions of amino acid sequence residues of the antibody.

Such amino acid variations are made based on relative similarity of amino acid side chain substituents such as hydrophobicity, hydrophilicity, charge, and size. According to analysis on sizes, shapes, and types of amino acid side chain substituents, it can be seen that arginine, lysine, and histidine are all positively charged residues; alanine, glycine, and serine have similar sizes; and phenylalanine, tryptophan, and tyrosine have similar shapes. Thus, based on these considerations, it can be said that arginine, lysine, and histidine; alanine, glycine, and serine; and phenylalanine, tryptophan, and tyrosine are biologically functional equivalents.

To introduce variations, the hydropathic index of amino acids may be considered. Each amino acid has been assigned a hydropathic index on the basis of their hydrophobicity and charge characteristics, which are as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). the hydropathic amino acid index is very important in conferring interactive biological function on a protein. It is known that certain amino acids may be substituted for other amino acids having a similar hydropathic index and still retain a similar biological activity. To introduce variations based on the hydropathic index, substitutions are made between amino acids that exhibit a hydropathic index difference of preferably within $\pm 2$, more preferably $\pm 1$, even more preferably $\pm 0.5$.

Meanwhile, it is also well known that substitution between amino acids having similar hydrophilicity values results in proteins having equivalent biological activity. As disclosed in U.S. Pat. No. 4,554,101, the following hydrophilicity values have been assigned to amino acid residues: arginine (+3.0); lysine (+3.0); aspartate (+3.0±1); glutamate (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). To introduce variations based on the hydrophilicity value, substitutions may be made between amino acids that exhibit a hydrophilicity value difference of preferably within $\pm 2$, more preferably $\pm 1$, even more preferably $\pm 0.5$.

Amino acid exchanges in proteins, which do not generally alter the activity of molecules, are known in the art. The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, and Gln/Glu.

Considering the above-described variations having biologically equivalent activity, it is interpreted that the binding molecules of the present invention also include a sequence showing substantial identity with the sequence set forth in the Sequence Listing.

In the present invention, the term "substantial identity" means a sequence showing at least 61% homology, more preferably 70% homology, even more preferably 80% homology, most preferably 90% homology, as determined by aligning the sequence of the present invention with any other sequence to correspond to each other as much as possible and analyzing the aligned sequence using an algorithm commonly used in the art. Alignment methods for sequence comparison are known in the art. Various methods and algorithms for alignment are accessible from NCBI Basic Local Alignment Search Tool (BLAST), NCBI (National Center for Biological Information), etc., and may be used in conjunction with sequencing programs, such as blastp, blasm, blastx, tblastn and tblastx, on the Internet. BLAST is available from http://www.ncbi.nlm.nih.gov/BLAST/. Sequence homology comparison methods using this program can be identified online (http://www.ncbi.nlm.nih.gov/BLAST/blast_help.html).

In the present invention, although the binding molecule, preferably the antibody, may be produced by a conventional method for producing an antibody, it may be produced by affinity maturation.

14

In the present invention, the "affinity maturation" refers to a process in which antibodies having increased affinity for an antigen are produced by activated B cells in the course of an immune response. For the purpose of the present invention, the affinity maturation allows antibodies or antibody fragments to be produced due to affinity maturation based on the principles of mutation and selection, in the same process as that occurring in nature.

In addition, in the present invention, the "monoclonal antibody" refers to an antibody molecule of a single molecular composition which is obtained from substantially the same antibody population, and exhibits single binding specificity and affinity for a particular epitope.

In the present invention, the "binding" or "specific binding" refers to the affinity of the antibody or antibody composition herein for an antigen. In antigen-antibody binding, the "specific binding" is distinguishable from non-specific background binding, typically in a case where a dissociation constant (Kd) is less than $1\times10\text{-}5$ M, less than $1\times10\text{-}6$ M, or less than $1\times10\text{-}7$ M. Specific binding can be detected by methods known in the art, such as ELISA, surface plasmon resonance (SPR), immunoprecipitation, and coprecipitation, which include an appropriate control that can distinguish between non-specific binding and specific binding.

The antibody or antigen-binding fragment of the present invention may exist as a multimer, such as a dimer, a trimer, a tetramer, or a pentamer, which includes at least part of antigen-binding capacity of a monomer. Such a multimer also includes a homomultimer or a heteromultimer. Antibody multimers contain a large number of antigen-binding sites, and thus have superior antigen-binding capacity as compared with monomers. Antibody multimers are also easily used to produce multifunctional (that is, bifunctional, trifunctional, tetrafunctional, or the like) antibodies.

In the present invention, the "multifunctional" refers to an antibody or antigen-binding fragment that has two or more activities or functions (for example, antigen-binding capacity, enzyme activity, and ligand- or receptor-binding capacity). For example, the antibody of the present invention may be bound to a polypeptide having enzymatic activity, such as luciferase, acetyltransferase, galactosidase, or the like. Multifunctional antibodies also include multivalent or multispecific (that is, bispecific, trispecific, or the like) forms of antibodies.

According to another embodiment of the present invention, there is provided a pharmaceutical composition for preventing or treating a brain and nervous system disease comprising, as an active ingredient, a chimeric antigen receptor (CAR) comprising an antigen-specific binding domain, a linking domain and a CD3 zeta ($\zeta$) signaling domain.

As used herein, the term "chimeric antigen receptor" or "CAR" refers to an engineered receptor comprising an extracellular antigen binding domain and an intracellular signaling domain. Although the most common type of CAR comprises a single chain variable fragment (scFv) derived from a monoclonal antibody fused to the transmembrane and intracellular domains of a T cell co-receptor, such as the CD3 zeta ($\zeta$) chain, the present invention is not limited to these domains. Rather, the "chimeric antigen receptor" or "CAR" as used herein refers to any receptor engineered to express any intracellular signaling molecule and any extracellular antigen binding domain fused or linked thereto. In the present invention, the binding domain may comprise a single chain variable fragment (scFv) capable of specifically recognizing the Lrig-1 protein. In the present invention, the term "single chain variable fragment" or "scFv" refers to a fusion protein of a variable heavy chain (VH) and a variable light chain (VL) of an antibody by a peptide linker between VL and VH.

In addition, in the present invention, the VH domain and the VL domain may be connected through a flexible linker. In the present invention, the flexible linker may be a glycine/serine linker of about 10 to 30 amino acids (e.g., 30, 25, 20, 19, 18, 17, 16, 15, 14, 13, 12, 11, 10, 9, 8, 7, 6, or 5 amino acids), preferably 15 amino acids in length. In the present invention, the linker length can act as an important determining site for chimeric antigen receptors, so a linker shorter than the above range can increase affinity, but can also cause intracellular multimer formation to impair CAR expression, while linkers longer than this range may reduce antigen affinity by moving the VL and VH CDRs farther apart in proximity.

The chimeric antigen receptor of the present invention may further comprise at least one of a hinge region (or spacer) and a signaling domain. In the present invention, the hinge region is a region connecting antigen-binding domain and the transmembrane domain, and is also called a "spacer," and has the purpose of extending the antigen-binding domain from the T cell membrane or the NK cell membrane. In the present invention, the hinge region can be obtained from any suitable sequence from any genus, including, for example, human or parts thereof, or may include CD8, CD28, 4-1BB, OX40, all or part of the CD3 zeta ($\zeta$) chain, T cell receptor $\alpha$ or $\beta$ chain, CD28, CD3$\varepsilon$, CD45, CD4, CD5, CD8, CD9, CD16, CD22, CD33, CD37, CD64, CD80, CD86, CD134, CD137, ICOS, CD154, functional derivatives thereof, or combinations thereof commonly used in the art, but is not limited thereto. In addition, the hinge region may comprise one selected from immunoglobulins (e.g., IgG1, IgG2, IgG3, IgG4, and IgD) without being limited to immunoglobulins, but is not limited thereto.

In the present invention, the signaling domain refers to a part of a chimeric antigen receptor found or engineered to be found inside a T cell. The signaling domain may or may not comprise a transmembrane that serves to fix the chimeric antigen receptor in the plasma membrane of T cells. The transmembrane domain and the signaling domain may be derived from the same protein (e.g., CD3 zeta ($\zeta$) molecule), or the transmembrane domain and the signaling domain may be derived from different proteins (e.g., transmembrane domain of CD28 and intracellular signaling domain of CD3 zeta ($\zeta$) molecule, or vice versa).

In the present invention, the transmembrane domain comprises, for example, T cell receptor $\alpha$ or $\beta$ chain, all or

part of the CD3 zeta (ζ) chain, CD28, CD3ε, CD45, CD4, CD5, CD8, CD9, CD16, CD22, CD33, CD37, CD64, CD80, CD86, CD134, CD137, ICOS, CD154, functional derivatives thereof, or combinations thereof, but is not limited thereto. The costimulatory domain may comprise 4-1BB (CD137), OX40, CD27, CD28, CD30, CD40, PD-1, CD2, CD7, CD258, natural killer group 2 member C (NKG2C), natural killer group 2 member (NKG2D), B7-H3, CD83, ICAM-1, ligands that bind to LFA-1 (CD11a/CD18) or ICOS, active fragments thereof, functional derivatives thereof, or a functional signaling domain derived from a polypeptide comprising a combination thereof, but is not limited thereto.

In the present invention, the signaling domain may comprise a functional signaling domain derived from polypeptides comprising all or part of CD3 zeta (ζ), common FcR gamma (FcER1G), FcgammaRIIIa, FcRbeta (Fc epsilon lip), CD3gamma, CD3delta, CD3 epsilon, CD79a, CD79b, DNAX-activating protein 10 (DAP10), DNAX-activating protein 12 (DAP12), an active fragment thereof, a functional derivative thereof, or a combination thereof, but is not limited thereto, and such signaling domains are known in the art.

The brain nervous system disease that the compositions provided by the present invention are intended to prevent, ameliorate, or treat may include neurodegenerative disease or a neuroinflammatory disease.

In the present invention, the "neurodegenerative disease" and the "neuroinflammatory disease" may be selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy, without being limited thereto.

Additionally, the compositions provided in this invention may be used as pharmaceutical compositions or food compositions, but are not limited thereto.

Meanwhile, in the prevention, "prevention" may include, without limitation, any act of blocking symptoms of a disease, or suppressing or delaying the symptoms, using the pharmaceutical composition of the present invention.

In addition, in the present invention "treatment" may include, without limitation, any act of ameliorating or beneficially altering symptoms of a disease, using the pharmaceutical composition of the present invention.

In the present invention, the pharmaceutical composition may be characterized by being in the form of capsules, tablets, granules, injections, ointments, powders, or beverages, and the pharmaceutical composition may be characterized by being intended for human subjects.

In the present invention, for use, the pharmaceutical composition may be formulated in the form of oral preparations such as powders, granules, capsules, tablets, and aqueous suspensions, preparations for external use, suppositories, and sterile injectable solutions, according to respective conventional methods, without being limited thereto. The pharmaceutical composition of the present invention may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The pharmaceutical composition of the present invention may be prepared in various dosage forms by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, examples of carriers, diluents, or excipients suitable for making preparations include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like. In addition, a filler, an anti-coagulant, a lubricant, a wetting agent, a fragrance, an emulsifier, a preservative, and the like may further be included.

The route of administration of the pharmaceutical composition of the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

In the present invention, the "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including the activity of a certain compound used, the patient's age, body weight, general health status, sex, diet, the time of administration, the route of administration, the rate of excretion, drug combination, and the severity of a certain disease to be prevented or treated. Although the dose of the pharmaceutical composition may vary depending on the patient's condition and body weight, the severity of disease, the drug form, the route of administration, and the duration of administration, it may be appropriately selected by those skilled in the art. The pharmaceutical composition may be administered at a dose of 0.0001 to 50 mg/kg/day or 0.001 to 50 mg/kg/day. Administration may be made once a day or several times a day. The dose is not intended to limit the scope of the invention in any way. The pharmaceutical composition according to the present invention may be formulated in the form of pills, sugar-coated tablets, capsules, liquids, gels, syrups, slurries, or suspensions.

A food composition containing the composition of the present invention as an active ingredient may be manufactured in various food products, such as beverages, gum, tea, vitamin complexes, powders, granules, tablets, capsules, candies, rice cakes, and bread. The food composition of the present invention is composed of plant extracts with minimal toxicity and side effects, so it can be safely used for long-term administration for preventive purposes.

When the composition of the present invention is included in a food composition, the amount can be added at a ratio of 0.1 to 50% of the total weight.

Here, when the food composition is manufactured in beverage form, there are no special restrictions other than containing the food composition at the specified ratio, and it may contain various flavorings or natural carbohydrates as additional components, similar to conventional beverages. That is, natural carbohydrates such as monosaccharides (e.g., glucose), disaccharides (e.g., fructose), polysaccharides (e.g., sucrose), dextrin, cyclodextrin, and other conventional sugars, as well as sugar alcohols such as xylitol, sorbitol, and erythritol, may be included. The flavorings may include natural flavorings (such as taumatin, stevia extracts (e.g., rebaudioside A, glycyrrhizin, etc.)) and synthetic flavorings (such as saccharin, aspartame, etc.).

In addition, the food composition of the present invention may contain various nutritional supplements, vitamins, minerals (electrolytes), flavorings (synthetic and natural), colorants, pectic acids and their salts, alginic acid and its salts, organic acids, protective colloidal thickeners, pH adjusters, stabilizers, preservatives, glycerin, alcohol, carbonating agents used in carbonated beverages, and the like.

These components may be used independently or in combination. The ratio of these additives is not particularly important, but it is generally selected within the range of 0.1 to approximately 50 parts by weight per 100 parts by weight of the composition of the present invention.

According to another embodiment of the present invention, for an individual requiring administration, the binding molecule provided by the present invention; the nucleic acid molecule encoding the binding molecule; the expression vector into which the nucleic acid molecule is inserted; the expression vector is introduced into host cell lines; or the antibody-drug conjugate (ADC) provided by the present invention is administered to the subject.

In the present invention, the "subject" is a subject suspected of developing brain nervous system disease. The subject suspected of developing brain nervous system disease refers to mammals, such as humans, mice, and domestic animals, which had or are likely to develop the disease in question. However, the subject includes, without limitation, any subject treatable with the effective substance provided by the present invention.

The brain and nervous system diseases treated by the method of the present invention may be neurodegenerative diseases or neuroinflammatory diseases and specific examples include stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy, without being limited thereto.

The method of the present invention may include administering the binding molecule provided by the present invention; the nucleic acid molecule encoding the binding molecule; the expression vector into which the nucleic acid molecule is inserted; the host cell line into which the expression vector is transfected; or the antibody-drug conjugate (ADC) provided by the present invention in a pharmaceutically effective amount.

The total daily dosage may be determined within the appropriate medical judgment range by the treating physician and may be administered in one or more divided doses. However, for the purposes of the present invention, the specific therapeutic effective dose for a particular patient may vary depending on the type and extent of the desired response, whether other formulations are used, the specific composition containing the active ingredient, the patient's age, weight, general health status, gender, diet, administration time, administration route, the secretion rate of the composition containing the active ingredient, the treatment duration, and various factors, including drugs used in combination with or concurrently with the specific composition, as well as similar factors well known in the pharmaceutical field, may vary depending on the specific circumstances.

Meanwhile, without being limited thereto, the method for preventing or treating the aforementioned brain neurological disorders may further include combination therapy comprising the administration of a compound or substance having therapeutic activity against one or more of the aforementioned disorders.

In the present invention, the term "combination" should be understood to mean simultaneous, individual, or sequential administration. In the case of sequential or individual administration, the interval between the administration of the second component should be such that the beneficial effects of the combination are not lost.

In the present invention, the dosage of the binding molecule or antibody-drug conjugate may be approximately 0.0001 μg to 500 mg per kilogram of patient body weight, but is not limited thereto.

According to another embodiment of the present invention, a diagnostic composition for immune-related diseases is provided, comprising a formulation for measuring the expression level of the Lrig-1 protein or its extracellular domain present on the surface of T cells; or the gene encoding the same, using the binding molecule.

The T cells of the present invention may be regulatory T cells.

The diagnostic composition of the present invention may include a formulation for measuring the expression level of the Lrig-1 protein or its extracellular domain present on the surface of regulatory T cells, such as activated regulatory T cells, i.e., regulatory T cells whose inhibitory function is activated; or the gene encoding the Lrig-1 protein, but is not limited thereto.

The diagnostic composition of the present invention may further include one or more proteins selected from the group consisting of CD25, TIGIT, LAG3, CTLA-4, GITR, OX40, ICOS, PD-1, TIM-3, CCR4, FR4, CD15s, PI-16, and Lrig-1 proteins present on the surface of T cells; or a composition for measuring the expression level of genes encoding the same. In this way, when measuring the expression level of various proteins present on the surface of T cells; or the expression level of genes encoding the same, compared to measuring the Lrig-1 protein or its extracellular domain alone; or the expression level of genes encoding the same, a significant synergistic effect can be achieved in diagnosing the target disease.

The composition of the present invention, which includes the expression levels of the Lrig-1 protein or its extracellular domain, and the expression levels of one or more proteins selected from the group consisting of CD25, TIGIT, LAG3, CTLA-4, GITR, OX40, ICOS, PD-1, TIM-3, CCR4, FR4, CD15s, PI-16, and Lrig-1 protein, may include one or more selected from a group consisting of antibodies, oligopeptides, ligands, PNA (peptide nucleic acid), and aptamers specific for the aforementioned proteins.

The composition of the present invention for measuring the expression level of one or more genes encoding the Lrig-1 protein (i.e., LAIR1) or the extracellular domain of the Lrig-1 protein, and one or more genes selected from the group consisting of CCD25, TIGIT, LAG3, CTLA-4, GITR, OX40, ICOS, PD-1, TIM-3, CCR4, FR4, CD15s, PI-16, and Lrig-1 protein, may include one or more selected from a group consisting of primers, probes, LNAs, and antisense nucleotides that specifically bind to the gene.

The "primers" of the present invention are fragments that recognize the target gene sequence, including forward and reverse primer pairs, but preferably primer pairs that provide analytical results with specificity and sensitivity. When the nucleic acid sequence of the primer is different from the non-target sequences present in the sample, and the primer contains a complementary primer binding site, it amplifies only the target gene sequence and does not cause non-specific amplification, thereby conferring high specificity.

The "probe" referred to in the present invention means a material capable of specifically binding to the target substance to be detected in the sample, and through such binding, a material capable of specifically confirming the presence of the target substance in the sample. The type of probe is not limited to materials commonly used in the art, but preferably includes PNA (peptide nucleic acid), LNA (locked nucleic acid), peptides, polypeptides, proteins, RNA, or DNA, and most preferably PNA. More specifically, the probes may include biological materials derived from or similar to biological organisms, or those manufactured ex vivo, such as: enzymes, proteins, antibodies, microorganisms, animal and plant cells and tissues, neurons, DNA, and RNA, where DNA includes cDNA, genomic DNA, and oligonucleotides, RNA includes genomic RNA, mRNA, and oligonucleotides, and proteins include antibodies, antigens, enzymes, peptides, and the like.

According to another embodiment of the present invention, the present invention relates to a diagnostic kit for brain nervous system diseases comprising the binding molecules provided by the present invention.

The kit of the present invention may be an RT-PCR kit, a DNA chip kit, an ELISA kit, a protein chip kit, a rapid kit, or an MRM (Multiple Reaction Monitoring) kit, but is not limited thereto.

The kit of the present invention may further include one or more other component compositions, solutions, or devices suitable for the analytical method.

The kit of the present invention may further include essential elements necessary for performing reverse transcription polymerase chain reaction. The reverse transcription polymerase chain reaction kit includes primer pairs specific for genes encoding marker proteins. The primer pairs are nucleotides having sequences specific to the nucleic acid sequence of the gene, for example, having a length of about 7 bp to 50 bp, or about 10 bp to 30 bp. The kit may also include primer pairs specific to the nucleic acid sequence of a control gene. The reverse transcription polymerase chain reaction kit may further include test tubes or other suitable containers, reaction buffers (with varying pH and magnesium concentrations), deoxyribonucleotides (dNTPs), enzymes such as Taq polymerase and reverse transcriptase, DNase, RNase inhibitors, DEPC-treated water (DEPC-water), sterile water, and other components.

The kit of the present invention may include essential elements required for performing DNA chip analysis. A DNA chip kit may include a substrate to which cDNA or oligonucleotides corresponding to a gene or its fragment are attached, and reagents, formulations, enzymes, etc., for preparing fluorescently labeled probes. Additionally, the substrate may include cDNA or oligonucleotides corresponding to a control gene or its fragment.

The kit of the present invention may include the essential elements required to perform ELISA. An ELISA kit includes antibodies specific to the protein. The antibodies are antibodies with high specificity and affinity for the marker protein and low cross-reactivity with other proteins, such as monoclonal antibodies, polyclonal antibodies, or recombinant antibodies. Additionally, the ELISA kit may include antibodies specific to a control protein. Furthermore, the ELISA kit may include reagents capable of detecting the conjugated antibody, such as labeled secondary antibodies, chromophores, enzymes (e.g., conjugated with antibodies), and their substrates, or other substances capable of binding to the antibody.

According to another embodiment of the present invention, the method provides information for diagnosing a disease by including a step of measuring the expression level of the Lrig-1 protein or its extracellular domain present on the surface of T cells in biological samples isolated from the target organism using the aforementioned binding molecules; or the expression level of the gene encoding the Lrig-1 protein.

The T cells of the present invention may be regulatory T cells.

The step of measuring the expression level of the present invention may further include measuring the expression level of one or more proteins selected from the group consisting of CD25, TIGIT, LAG3, CTLA-4, GITR, OX40, ICOS, PD-1, TIM-3, CCR4, FR4, CD15s, PI-16, and Lrig-1 proteins present on the surface of T cells; or the expression level of genes encoding these proteins. In this way, when the expression level of various proteins present on the surface of T cells; or the expression level of genes encoding these proteins is additionally measured, a significant synergistic effect can be achieved in diagnosing the target disease compared to when the Lrig-1 protein; or the gene encoding this protein is measured alone.

The "target subject" referred to in the present invention is an individual whose status regarding the onset of the disease is uncertain, meaning an individual with a high likelihood of developing the disease.

The "biological sample" referred to in the present invention means any material, biological fluid, tissue, or cell obtained from or derived from an organism, such as whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, or cerebrospinal fluid, but is not limited thereto.

The Lrig-1 protein of the present invention; or its extracellular domain may be expressed on the cell surface of T cells, particularly regulatory T cells, such as activated regulatory T cells, i.e., effector T cells, where the inhibitory function of activated regulatory T cells is activated.

The Lrig-1 protein or its extracellular domain of the present invention, and CD25, TIGIT, LAG3, CTLA-4, GITR, OX40, ICOS, PD-1, TIM-3, CCR4, FR4, CD15s, PI-16, and Lrig-1 protein, the expression levels of one or more proteins selected from the group consisting of the above proteins can be measured or compared using methods such as protein chip analysis, immunoassay, ligand binding assay, MALDI-TOF (Matrix-Assisted Laser Desorption/Ionization Time-of-Flight Mass Spectrometry) analysis, SELDI-TOF (Surface-Enhanced Laser Desorption/Ionization Time of Flight Mass Spectrometry) analysis, radioimmunoassay, radioimmunodiffusion, octreotide immunodiffusion, rocket immunodiffusion, tissue immunofluorescence, complement fixation assay, two-dimensional electrophoresis analysis, liquid chromatography-mass spectrometry (LC-MS), LC-MS/MS (Liquid chromatography-Mass Spectrometry/Mass Spectrometry), Western blotting, or ELISA (Enzyme-linked immunosorbent assay), among others, but this list is not exhaustive.

The present invention relates to a method for identifying cells expressing the Lrig-1 protein or its extracellular domain, or a protein encoded by a gene encoding the Lrig-1 protein, and cells expressing one or more proteins selected from the group consisting of CD25, TIGIT, LAG3, CTLA-4, GITR, OX40, ICOS, PD-1, TIM-3, CCR4, FR4, CD15s, PI-16, and Lrig-1 protein, and the expression levels of the aforementioned genes can be measured using analytical methods such as reverse transcription polymerase chain reaction (RT-PCR), Competitive RT-PCR, Real-time RT-PCR, RNase protection assay (RPA), Northern blotting, or DNA chips, among others, but is not limited to these.

The present invention may include a step of predicting a high likelihood of the onset of an immune-related disease when the expression level of the Lrig-1 protein or its extracellular domain; or the gene encoding the Lrig-1 protein, measured in biological samples of the subject of the present invention, is reduced compared to a normal control group.

The present invention may further include a step of predicting a high likelihood of the onset of an immune-related disease when the expression level of one or more proteins selected from the group consisting of CD25, TIGIT, LAG3, CTLA-4, GITR, OX40, ICOS, PD-1, TIM-3, CCR4, FR4, CD15s, PI-16, and Lrig-1 proteins present on the surface of T cells; or the expression level of genes encoding these proteins is altered (increased or decreased) compared to a normal control group, the method may further include a step of predicting a high likelihood of developing a disease.

In another specific example of the present invention, the step of predicting a high likelihood of developing a disease may include cases where the expression level of the Lrig-1 protein; or the gene encoding it is reduced compared to a normal control group.

Furthermore, in the present invention, when the expression level of the Lrig-1 protein or its extracellular domain;

or the gene encoding the Lrig-1 protein, measured in the biological sample of the subject of the present invention, is measured and predicted or diagnosed as having a high likelihood of developing a disease, the step of administering a drug for the disease to the subject of the present invention may be further included.

In one embodiment of the present invention, an epitope of the Lrig-1 (leucine-rich and immunoglobulin-like domains 1) protein is provided, selected from a group consisting of polypeptides comprising the amino acid sequences represented by SEQ ID NOs:22, 25, 28, 30, 33, 38, 41, 43, 44, 47, 51, and 60, is provided.

In another embodiment of the present invention, the polypeptide comprises an epitope selected from the group consisting of polypeptides comprising amino acid sequences represented by SEQ ID NOs: 21, 23, 24, 26, 27, 29, 31, 32, 34 to 40, 42, 45, 46, 48 to 50, 52 to 59, or 61 to 69.

In one embodiment of the present invention, a nucleic acid molecule encoding the epitope is provided.

In one embodiment of the present invention, an expression vector containing the nucleic acid molecule is provided.

In one embodiment of the present invention, the expression vector is provided in a host cell line that has been genetically modified.

In one embodiment of the present invention, a binding molecule specifically binding to at least one epitope selected from a group consisting of polypeptides composed of an amino acid sequence represented by SEQ ID NOs: 21 to 69 is provided.

In another embodiment of the present invention, the binding molecule is provided as an antibody or an antigen-binding fragment.

In another embodiment of the present invention, the antibody is a chimeric antibody, humanized antibody, bivalent, bispecific molecules, minibodies, domain antibodies, bispecific antibodies, antibody mimics, diabodies, triabodies, tetrabodies, or fragments thereof.

In one embodiment of the present invention, a chimeric antigen receptor (CAR) comprising an antigen-specific binding domain, a linker domain, and a CD3 $\zeta$ signaling domain, the antigen-specific binding domain is an antigen-specific binding domain that specifically binds to at least one epitope selected from a group consisting of polypeptides composed of amino acid sequences represented by SEQ ID NOs: 21 to 69; and a chimeric antigen receptor comprising a fusion protein including an immunoglobulin Fc region.

In one embodiment of the present invention, an antibody-drug conjugate comprising a binding molecule and a drug is provided.

In one embodiment of the present invention, a pharmaceutical composition for the prevention or treatment of a brain nervous system disease is provided, comprising as an active ingredient one or more of the binding molecule, the chimeric antigen receptor, and the antibody-drug conjugate.

In another embodiment of the present invention, the pharmaceutical composition is provided for the prevention or treatment of neurodegenerative diseases or neuroinflammatory diseases.

In another embodiment of the present invention, the neurodegenerative disease or neuroinflammatory disease is stroke, dementia, Alzheimer's disease, Parkinson's disease, Huntington's disease, Niemann-Pick disease, prion disease, Creutzfeldt-Jakob disease, frontotemporal dementia, Lewy body dementia, amyotrophic lateral sclerosis (ALS), paraneoplastic syndrome, cortical basal degeneration, multiple system atrophy, progressive supranuclear palsy, neurological autoimmune diseases, spinocerebellar ataxia, inflammatory and neuropathic pain, cerebrovascular diseases, spinal cord injury, and tauopathy, comprising at least one selected therefrom, for the prevention or treatment of neurodegenerative diseases or neuroinflammatory diseases.

In one embodiment of the present invention, a method for screening a binding molecule, such as an antibody or a fragment thereof, that specifically binds to the epitope.

In one embodiment of the present invention, a method for screening the epitope of the Lrig-1 protein using the binding molecule is provided.

In one embodiment of the present invention, a method of binding the binding molecule, which is an antibody or an antigen-binding fragment thereof, to the epitope.

In one embodiment of the present invention, a binding molecule that specifically binds to the Lrig-1 (leucine-rich and immunoglobulin-like domains 1) protein, comprising an antibody or a fragment thereof, wherein: (a) the heavy chain variable region of the binding molecule comprises: the heavy chain CDR1 represented by SEQ ID NO: 7; the heavy chain CDR2 represented by SEQ ID NO: 8; and the heavy chain CDR3 represented by SEQ ID NO: 9; or (b) the variable region of the light chain of the binding molecule comprises: the light chain CDR1 represented by SEQ ID NO: 10; the light chain CDR2 represented by SEQ ID NO: 11; and the light chain CDR3 represented by SEQ ID NO: 12; the binding molecule provides a binding molecule that specifically binds to an epitope comprising at least one polypeptide selected from a group consisting of polypeptides composed of amino acid sequences represented by SEQ ID NOs: 21 to 69.

In another embodiment of the present invention, the heavy chain variable region of the binding molecule is represented by SEQ ID NO: 13, or the light chain variable region is represented by SEQ ID NO: 14.

In another embodiment of the present invention, the binding molecule is provided wherein the heavy chain is represented by SEQ ID NO: 15 or 17, and the light chain is represented by SEQ ID NO: 16 or 18.

Advantageous Effects of Invention

The antibody or antigen-binding fragment that specifically binds to the epitope of the Lrig-1 protein according to the present invention specifically binds to the epitope of the present invention present on regulatory T cells, thereby regulating the function of the regulatory T cells, and maintaining or enhancing the activity of effector T cells, making it highly effective for the prevention, improvement, or treatment of various diseases.

Brief Description of Drawings

FIG. 1 illustrates an experimental design for identifying therapeutic effects of monoclonal antibodies on Alzheimer's, according to an embodiment of the present invention.

FIG. 2 illustrates a photograph of results obtained with a fluorescence microscope by staining the brain cortex and hippocampal tissue of the mouse with Thioflavin S (ThS) obtained after each treatment of Alzheimer's-induced mice with the monoclonal antibody according to an embodiment of the present invention.

FIG. 3 illustrates a graph showing the results of calculating the ThS+ area ratio (%) relative to normal mice after staining the brain cortex and hippocampal tissue of the mouse with Thioflavin S (ThS) obtained after each treatment of Alzheimer's-induced mice with the monoclonal antibody according to an embodiment of the present invention.

FIG. 4 illustrates a graph showing changes in spontaneous alternation (%) values as a result of Y-maze test after each treatment of Alzheimer's-induced mice with the monoclonal antibody according to an embodiment of the present invention.

FIG. 5 illustrates a graph showing changes in preference values as a result of a novel object recognition experiment after each treatment of Alzheimer's-induced mice with the monoclonal antibody according to an embodiment of the present invention.

FIG. 6 illustrates a graph showing changes in the time taken to reach the position of the platform as a result of the water maze experiment after each treatment of Alzheimer's-induced mice with the monoclonal antibody according to an embodiment of the present invention.

FIG. 7 illustrates a graph showing changes in the number of times the Alzheimer's-induced mice passed the target as a result of the water maze experiment after each treatment of Alzheimer's-induced mice with the monoclonal antibody according to an embodiment of the present invention.

FIG. 8 illustrates a graph showing calculated changes in the time taken to reach the position of the platform during the water maze experiment after each treatment of Alzheimer's-induced mice with the monoclonal antibody according to an embodiment of the present invention.

FIG. 9 illustrates an experimental design of Alzheimer's-induced 5xFAD mice and 6xTg mice for identifying therapeutic effects of monoclonal antibodies on Alzheimer's, according to an embodiment of the present invention.

FIG. 10 illustrates a graph showing changes in spontaneous alternation (%) values as a result of Y-maze test after each treatment of Alzheimer's-induced 5xFAD mice and 6xTg mice with the monoclonal antibody according to an embodiment of the present invention.

FIG. 11 illustrates a graph showing changes in preference values as a result of a novel object recognition experiment after each treatment of Alzheimer's-induced 6xTg mice with the monoclonal antibody according to an embodiment of the present invention.

FIG. 12 illustrate a graph showing changes in retention time as a result of a passive avoidance experiment after each treatment of Alzheimer's-induced 5xFAD mice and 6xTg mice with the monoclonal antibody according to an embodiment of the present invention.

FIG. 13 illustrate the trypsin CL-MS labeled peptide percentage (tryptic peptide positions).

FIG. 14 illustrate the trypsin CL-MS labeled peptide% (point tryptic peptide positions).

FIG. 15 illustrate trypsin CL-MS labeled peptides% (tryptic peptide positions & labeling residue).

FIG. 16 illustrate the chymotrypsin CL-MS labeled peptide % (chymotrypsin peptide positions).

FIG. 17 illustrate the chymotrypsin CL-MS analysis labeling peptide% (chymotryptic peptide positions & labeling residue).

FIG. 18 illustrate the CL-MS histogram.

FIG. 19 illustrate the trypsin XL-MS graph (detected peptides).

FIG. 20 illustrate the trypsin XL-MS graph (decreased peptides).

FIG. 21 illustrate the chymotrypsin XL-MS graph (detected peptides).

FIG. 22 illustrate the chymotrypsin XL-MS graph (decreased peptides).

FIG. 23 illustrate the XL-MS histogram.

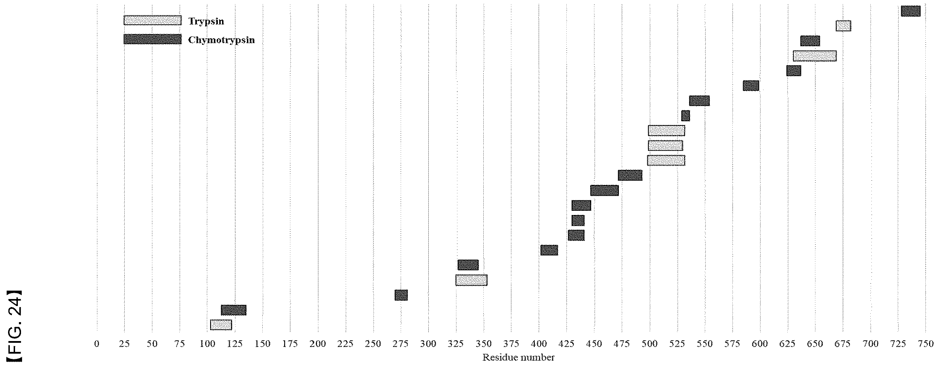

FIG. 24 illustrate the affinity-MS histogram.

FIG. 25 illustrate the epitope mapping histogram.

Detailed Description of Invention

Hereinafter, the present invention will be described in more detail by way of examples. These examples are only for describing the present invention in more detail, and it will be apparent to those skilled in the art that according to the gist of the present invention, the scope of the present invention is not limited by these examples.

## Examples

### [Production Examples] Production of monoclonal antibodies specific to Lrig-1 protein

Antibodies specific for the Lrig-1 protein according to the present invention were produced. The present antibodies were not produced by specifying a certain epitope, but were produced as antibodies capable of binding to any site on the Lrig-1 protein.
In order to produce the antibodies, cells expressing the Lrig-1 protein were produced. More specifically, a DNA fragment corresponding to SEQ ID NO: 2 and pcDNA (hygro) were cleaved with a cleavage enzyme, incubated at 37°C, and ligated to produce pcDNA into which a DNA sequence of the Lrig-1 protein is inserted. The thus produced pcDNA into which SEQ ID NO: 2 is inserted was introduced, through transfection, into T cells, so that the Lrig-1 protein is allowed to be expressed on the surface of the T cells.
Light and heavy chain amino acid sequences capable of binding to Lrig-1 expressed on the cell surface were selected from the Human scFv library.
The selected heavy chain and light chain amino acid sequences were fused with the heavy chain constant region and light chain constant region to produce a monoclonal antibody. The sequence of the monoclonal antibody is shown in Table 1 below.

[Table 1]

| Classification | Clone | Location | Amino acid sequence | Sequence information |
|---|---|---|---|---|
| Production Example 1 | GTC310-01 mouse antibody | Heavy chain | EVQLLESGGGLVQPGGSLRLSCAASG FTFS NYAMS WVRQAPGKGLEWVS VISHGGGSTYYADSVKG RFTISRDNSKNTLYLQMNSLRAEDTA VYYCAR VISNCHLGVCYYSNGMDV WGQGTLVTVSSAKTTAPSVYPLAPVC GDTTGSSVTLGCLVKGYFPEPVTLTW NSGSLSSGVHTFPAVLQSDLYTLSSSV TVTSSTWPSQSITCNVAHPASSTKVD | SEQ ID NO: 15 |
| | | | KKIEPRGPTIKPCPPCKCPAPNLLGGPS VFIFPPKIKDVLMISLSPIVTCVVVDVS EDDPDVQISWFVNNVEVHTAQTQTH REDYNSTLRVVSALPIQHQDWMSGK EFKCKVNNKDLPAPIERTISKPKGSVR APQVYVLPPPEEEMTKKQVTLTCMV TDFMPEDIYVEWTNNGKTELNYKNT EPVLDSDGSYFMYSKLRVEKKNWVE RNSYSCSVVHEGLHNHHTTKSFSRTP GK | |
| | | Light chain | QLVLTQPPSVSAAPGQRVSISC SGSSSNIGDNYVS WYQQVPGSAPKLLIY DNNKRPS GIPDRFSASKSGTSATLGITGLQTGDE ADYYCAT WDGSLSAGRV FGGGTKLTVL RADAAPTVSIFPPSSEQLTSGGASVVC FLNNFYPKDINVKWKIDGSERQNGVL NSWTDQDSKDSTYSMSSTLTLTKDEY ERHNSYTCEATHKTSTSPIVKSFNRNE C | SEQ ID NO: 16 |

EP 4 678 655 A1

| Classification | Clone | Location | Amino acid sequence | Sequence information |
|---|---|---|---|---|
| Production Example 2 | GTC310-01 humanized antibody | Heavy chain | EVQLLESGGGLVQPGGSLRLSCAASG FTFS NYAMS WVRQAPGKGLEWVS VISHGGGSTYYADSVKG RFTISRDNSKNTLYLQMNSLRAEDTA VYYCAR VISNCHLGVCYYSNGMDV WGQGTLVTVSSASTKGPSVFPLAPSS KSTSGGTAALGCLVKDYFPEPVTVSW NSGALTSGVHTFPAVLQSSGLYSLSSV VTVPSSSLGTQTYICNVNHKPSNTKV DKKVEPKSCDKTHTCPPCPAPELLGG PSVFLFPPKPKDTLMISRTPEVTCVVV DVSHEDPEVKFNWYVDGVEVHNAKT KPREEQYNSTYRVVSVLTVLHQDWL NGKEYKCKVSNKALPAPIEKTISKAK GQPREPQVYTLPPSREEMTKNQVSLT CLVKGFYPSDIAVEWESNGQPENNYK TTPPVLDSDGSFFLYSKLTVDKSRWQ QGNVFSCSVMHEALHNHYTQKSLSLS PGK | SEQ ID NO: 17 |
| | | Light chain | QLVLTQPPSVSAAPGQRVSISC SGSSSNIGDNYVS WYQQVPGSAPKLLIY DNNKRPS GIPDRFSASKSGTSATLGITGLQTGDE ADYYCAT WDGSLSAGRV FGGGTKLTVL RTVAAPSVFIFPPSDEQLKSGTASVVC LLNNFYPREAKVQWKVDNALQSGNS QESVTEQDSKDSTYSLSSTLTLSKADY EKHKVYACEVTHQGLSSPVTKSFNRG EC | SEQ ID NO: 18 |
| Heavy chain | | CDR1 | NY AMS | SEQ ID NO: 7 |
| | | CDR2 | VISHGGGSTY YADSVKG | SEQ ID NO: 8 |
| | | CDR3 | VISNCHLGVC YYSNGMDV | SEQ ID NO: 9 |
| Light chain | | CDR1 | SGSSSNIGDN YVS | SEQ ID NO: |
| | | | 10 | |
| | CDR2 | DNNKRPS | SEQ ID NO: 11 | |

(continued)

| Classification | Clone | Location | Amino acid sequence | Sequence information |
|---|---|---|---|---|
|  | CDR3 | WDGSLSAGRV | SEQ ID NO: 12 |  |

**[Example 1] Evaluation of therapeutic ability of antibody according to present invention against Alzheimer's disease - 5xFAD mice**

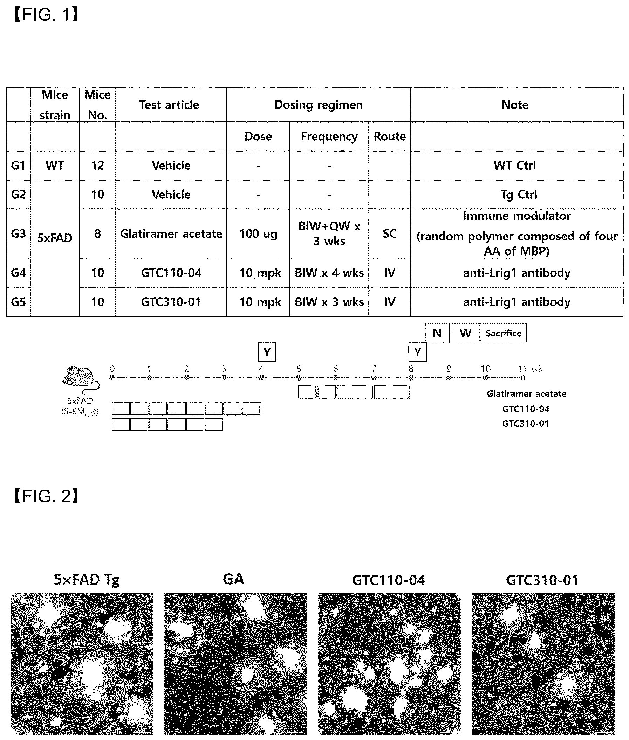

In order to evaluate the therapeutic ability of the antibody according to the present invention against Alzheimer's disease, groups were designed as shown in FIG. 1. Specifically, 5- to 6-month-old male 5xFAD mice with induced Alzheimer's disease were intravenously injected with GTC310-01 mouse antibody at an amount of 10mpk each for 3 weeks. However, glatiramer acetate (GA, Copaxone®) was subcutaneously injected at an amount of 100 µg for 3 weeks, or the H6 antibody (GTC110-04) shown in Table 2 was intravenously injected in an amount of 10mpk for 4 weeks as a positive control.

[Table 2]

| Clone | Location | Amino Acid Sequence | SEQ ID NO |
|---|---|---|---|
| H6 antibody (GTC11 0-04) | Heavy chain | EVQLLESGGGLVQPGGSLRLSCAASGFTFSNYAMSWVRQAPGKG LEWVSVISHGGGSTYYADSVKGRFTISRDNSKNTLYLQMNSLRA EDTAVYYCARVISNCHLGVCYYSNGMDVWGQGTLVTVSSTTAPS VYPLAPVCGDTTGSSVTLGCLVKGYFPEPVTLTWNSGSLSSGVH TFPAVLQSDLYTLSSSVTVTSSTWPSQSITCNVAHPASSTKVDK KIEPRGPTIKPCPPCKCPAPNLLGGPSVFIFPPKIKDVLMISLS PIVTCVVVDVSEDDPDVQISWFVNNVEVHTAQTQTHREDYNSTL RVVSALPIQHQDWMSGKEFKCKVNNKDLPAPIERTISKPKGSVR APQVYVLPPPEEEMTKKQVTLTCMVTDFMPEDIYVEWTNNGKTE LNYKNTEPVLDSDGSYFMYSKLRVEKKNWVERNSYSCSVVHEGL HNHHTTKSFSRTPGK | SEQ ID NO: 19 |

(continued)

| Clone | Location | Amino Acid Sequence | SEQ ID NO |
|---|---|---|---|
| | Light chain | QSVLTQPPSASGTPGQRVTISCSGSSSNIGNNDVYWYQQLPGTA<br><br>PKLLIYSDSQRPSGVPDRFSGSKSGTSASLAISGLRSEDEADYY<br><br>CGTWDYSLSGYVFGGGTKLTVLRTVAAPTVSIFPPSSEQLTSGG<br>ASVVCFLNNFYPKDINVKWKIDGSERQNGVLNSWTDQDSKDSTY<br><br>SMSSTLTLTKDEYERHNSYTCEATHKTSTSPIVKSFNRNEC | SEQ ID NO: 20 |

### 1. Amyloid-β plaque reducing effect

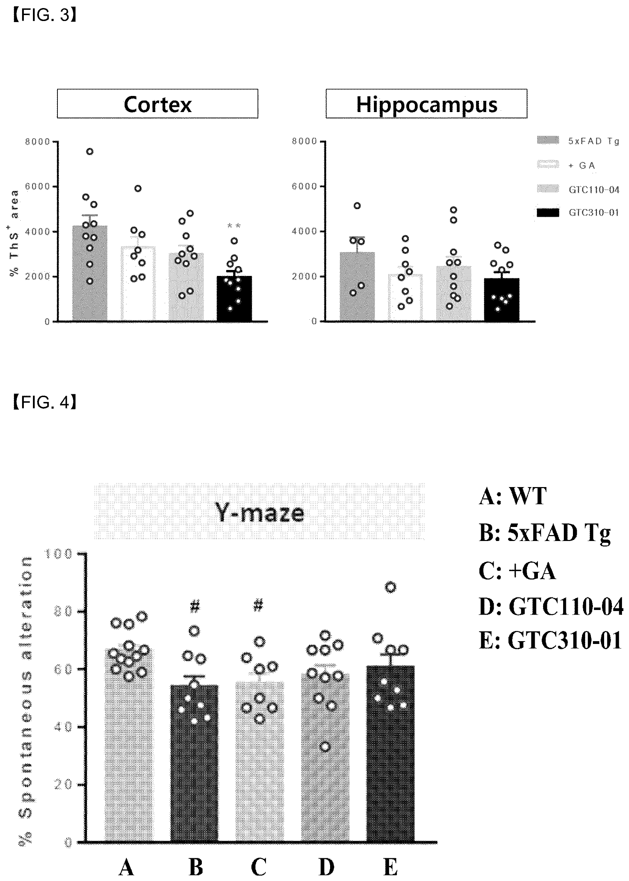

For immunohistological analysis, the above-treated mice were euthanized, and the cortex and hippocampus tissues were obtained and fixed in 4% paraformaldehyde (pH 7.4) for 16 hours. The fixed cortex and hippocampus tissues were immersed in 30% sucrose for cryoprotection and sliced to a thickness of 35 μm. To visualize Aβ plauqes, the sliced brain sections were stained with thioflavin S (ThS) for 7 minutes. Thioflavin S was purchased from Sigma-Aldrich (catalog number T-1892). Thioflavin S (ThS) at 500 μM was dissolved in 50% ethanol. After successive washes with 100%, 95% and 70% ethanol, the sections were transferred to PBS. Relatively thick free-floating brain slices were incubated with goat anti-GFAP antibody for 7 days at 4°C for immunofluorescence. Then, the sections were incubated overnight at 4°C with Alexa Fluor 594-conjugated donkey anti-goat IgG (1:200, Abcam, ab150132). Nuclear counterstaining was performed using 4'6'-damiino-2-phenylindole dihydrochloride hydrate (DAPI, 1 mg/mL, 1:2000, Sigma). Images were taken with a Leica DM2500 fluorescence microscope, and the results are shown in FIG. 2. Additionally, the ThS+ area ratio (%) relative to normal mice was calculated using the ImageJ software program, and the results are shown in FIG. 3.

As shown in FIGS. 2 and 3, although the ThS+ area significantly increased in Alzheimer's-induced mice, when the GTC310-01 antibody according to the present invention was administered, the ThS+ area was significantly reduced, especially in the cortex, thus showing that the effect of reducing amyloid-β plaques was excellent.

### 2. Y-maze test

For the Y-maze test, a Y-shaped maze was used which had three identical arms having a length of 40 cm (with a 15-cm high wall) at an angle of 120 degrees from each other. This experiment was a behavioral experiment using the rodent's instinctive exploring habit and was based on the fact that there is a high possibility of exploring a new area. The higher the degree that the test animal remembered the arm it had just explored and tried not to enter the same arm, the higher the animal's memory level. An exploring time of 8 minutes was given to each individual, and the final results were expressed as spontaneous alteration (%) values in FIG. 4. The spontaneous alteration (%) value was calculated by Expression 1. Here, behavioral patterns were analyzed using SMART VIDEO TRACKING Software (Panlab, USA).

[Equation 1]

$$\text{Spontaneous alteration (\%)} = \frac{number\ of\ triplet}{total\ arm\ entry - 2}$$

As shown in FIG. 4, in this experiment for measuring the relative frequency at which the test animal perceives the surrounding clues and sequentially enters the maze, it was found that in the case where the mice with induced Alzheimer's disease were administered with the GTC310-01 antibody according to the present invention, the mice had a spontaneous alteration value at a level similar to that of a normal control.

**3. Novel Object Recognition**

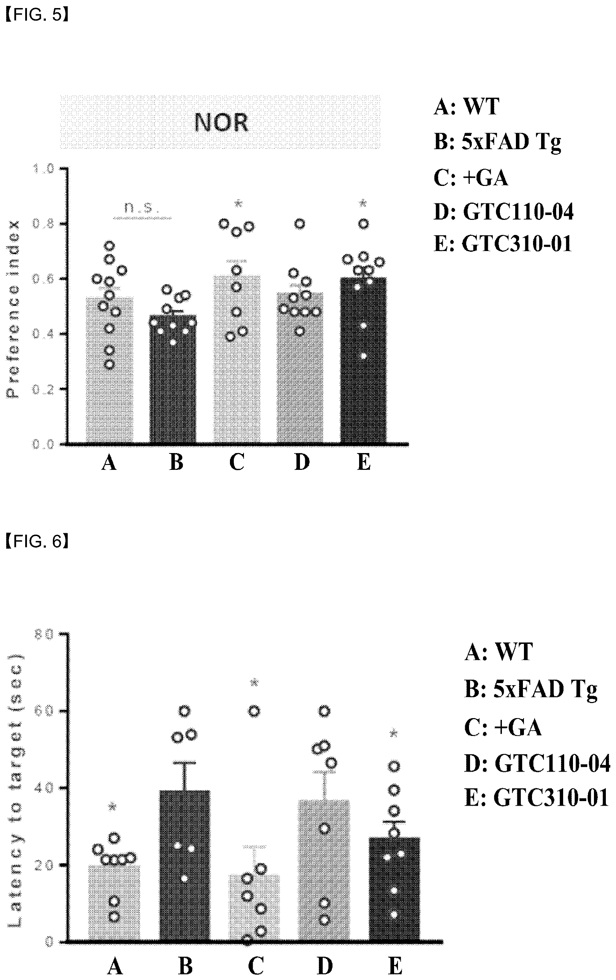

The novel object recognition test was performed to evaluate memory using two different objects in a 40 cm $\times$ 40 cm acrylic cage. After causing the test animal to acclimatize to the inside of the acrylic cage, two objects were placed at certain locations and the animal was allowed to perceive the objects freely. Then, the time for exploring each object was measured. 24-hour delay was given to each individual, and only one object was changed to another at the same location. Here, in a case where the animal perceives the changed object as a novel object and spends a longer exploring time, it may be determined that the animal has better memory. In a case where the animal does not remember the objects that it explored 24 hours before, the animal fails to distinguish between the novel object and the old object, and thus there is a possibility that the animal explores equally both objects. The animal was allowed to explore freely for a total of 10 minutes, and the result was expressed as a preference index (which equals novel object exploring time/total exploring time) in FIG. 5. Here, the analysis was performed using SMART VIDEO TRACKING Software (Panlab, USA).

As shown in FIG. 5, the preference index for a new object was lower in the mice with induced Alzheimer's disease compared with the normal control. However, when the GTC310-01 antibody according to the present invention was administered, the preference value was higher than that of the normal control.

**4. Water maze test**

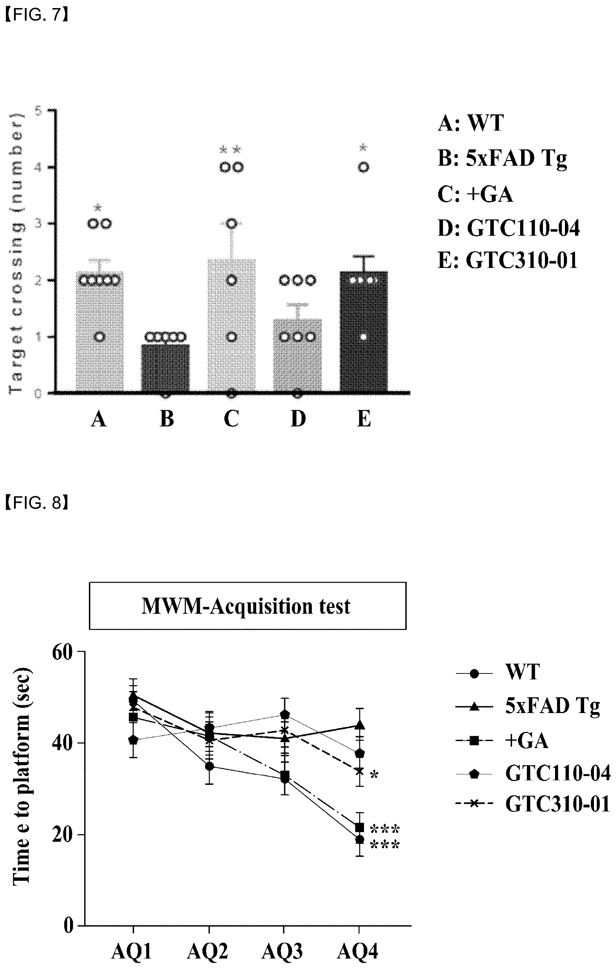

The water maze test was conducted based on the method devised by Morris. A stainless-steel pool (90 cm in diameter, 50 cm in height) was filled with water (22 $\pm$ 1°C) so that the height of the water surface was 30 cm. A hidden platform (5 cm in diameter) was placed 1 cm below the water surface. On day 1 of evaluation, 3 to 4 training sessions were allowed. A total swimming time per individual was set to 60 seconds; and the individual, who found the platform within 60 seconds, was allowed to stay on the platform for 10 seconds to induce memory. The individual, who was unable to find the platform even after 60 seconds, was manually guided to the platform and allowed to stay on the platform for 10 seconds, wherein the time to escape was set to 60 seconds. On day 6 after the training, a probe test was conducted to calculate spatial perception (time taken to reach the location of the platform (latency to target, sec)) and the number of times it crossed the target (target crossing numbers, number), the results of which are shown in FIGS. 6 and 7, and an acquisition test was conducted to calculate the time taken to reach the platform (time to platform, sec), the result of which is shown in FIG. 8. The analysis was performed using SMART VIDEO TRACKING Software (Panlab, USA). In interpreting the results, the individual included in the exclusion criteria was set as an individual who moves around a specific area without exploration through swimming.

As shown in FIGS. 6 to 8, when the GTC310-01 antibody according to the present invention was administered, the number of times it crossed the target increased significantly, and the time taken to reach the platform significantly decreased.

Through these experiments, it was found that the antibody according to the present invention had a prophylactic, ameliorating, or therapeutic effect on brain and nervous system diseases.

**[Example 2] Evaluation of therapeutic effect of antibodies according to the present invention for Alzheimer's - 6xTg mice**

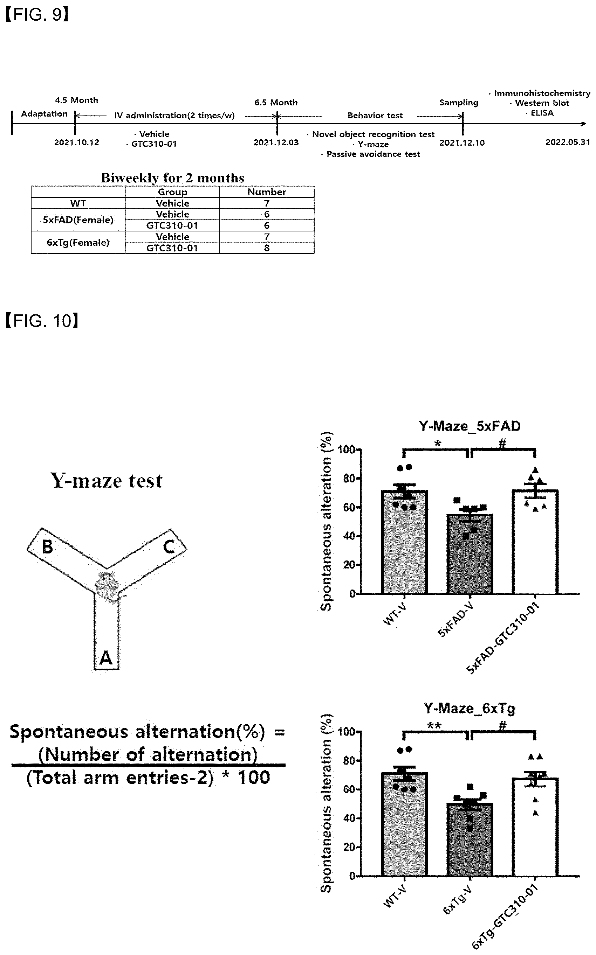

In order to evaluate the therapeutic ability of the antibody according to the present invention against Alzheimer's disease, groups were designed as shown in FIG. 9. Specifically, female 5xFAD mice with induced Alzheimer's disease and female 6xTg mice with induced Alzheimer's disease that underwent an adaptation period of 4.5 months were intravenously injected with GTC310-01 mouse antibody of Preparation Example 1 at an amount of 10mpk for 2 months. However, glatiramer acetate (GA, Copaxone®) was intravenously injected at an amount of 100 μg for 2 months as a positive control. After 2 months, the Y-maze test, novel object recognition experiment, and passive avoidance test were conducted.

### 1. Characteristics of 6xTg mice

Although the 5xFAD mice is widely used as an Alzheimer's-induced model, thee 6xTg mice used in the following experiment is a mouse model in which not only amyloid-$\beta$ (A$\beta$42) but also tau protein (MAPT) is mutated and accumulated, and thus is a model that is closer to a human disease model than the 5xFAD mouse, thus making it an ideal animal model for neurological diseases including Alzheimer's.

### 2. Y-maze test

For the Y-maze test, a Y-shaped maze was used which had three identical arms having a length of 40 cm (with a 15-cm high wall) at an angle of 120 degrees from each other. This experiment was a behavioral experiment using the rodent's instinctive exploring habit and was based on the fact that there is a high possibility of exploring a new area. The higher the degree that the test animal remembered the arm it had just explored and tried not to enter the same arm, the higher the animal's memory level. An exploring time of 8 minutes was given to each individual, and the final results were expressed as spontaneous alteration (%) values in FIG. 10. The spontaneous alteration (%) value was calculated by Expression 1. Here, behavioral patterns were analyzed using SMART VIDEO TRACKING Software (Panlab, USA).

$$[\text{Equation 1}]$$

$$\text{Spontaneous alteration (\%)} = \frac{number\ of\ triplet}{total\ arm\ entry - 2}$$

As shown in FIG. 10, in this experiment for measuring the relative frequency at which the test animal perceives the surrounding clues and sequentially enters the maze, it was found that in the case where the mice with induced Alzheimer's disease were administered with the GTC310-01 antibody according to the present invention, the mice had a spontaneous alteration value at a level similar to that of a normal control. Furthermore, mice of the 6xTg model, which is more similar to the human disease model, had a spontaneous alteration value more similar to that of the normal control group than the 5xFAD mouse.

### 3. Novel Object Recognition

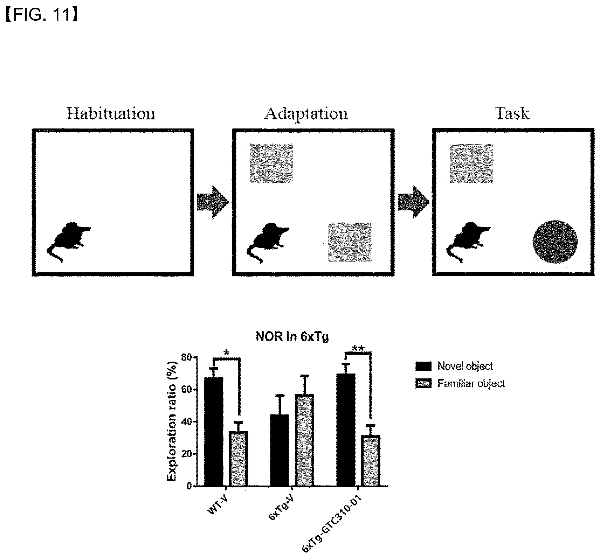

The novel object recognition test was performed to evaluate memory using two different objects in a 40 cm $\times$ 40 cm acrylic cage. After causing the test animal to acclimatize to the inside of the acrylic cage, two objects were placed at certain locations and the animal was allowed to perceive the objects freely. Then, the time for exploring each object was measured. 24-hour delay was given to each individual, and only one object was changed to another at the same location. Here, in a case where the animal perceives the changed object as a novel object and spends a longer exploring time, it may be determined that the animal has better memory. In a case where the animal does not remember the objects that it explored 24 hours before, the animal fails to distinguish between the novel object and the old object, and thus there is a possibility that the animal explores equally both objects. The animal was allowed to explore freely for a total of 10 minutes, and the result was expressed as a preference index (which equals novel object exploring time/total exploring time) in FIG. 11. Here, the analysis was performed using SMART VIDEO TRACKING Software (Panlab, USA).

As shown in FIG. 11, the preference index for a new object was lower in the mice with induced Alzheimer's disease compared with the normal control. However, when the GTC310-01 antibody according to the present invention was administered, the preference value was higher than that of the normal control in the 6xTg model, which is closer to the human disease model.

### 4. Passive Avoidance Test

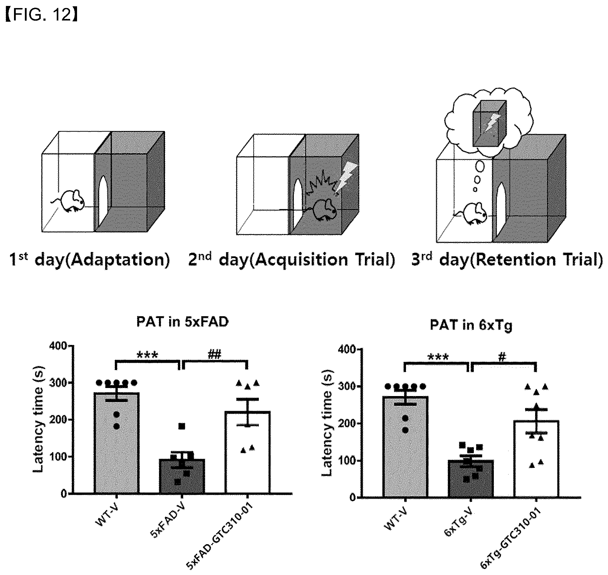

The passive avoidance test experimental equipment is divided into two zones, a bright chamber with lighting and a dark chamber, and the floor is made of wire mesh. On the first day, the mice were allowed to freely move and adapted. The following day, each of the 5xFAD mice and 6xTg mice were adapted in a lighted chamber without the lights on for 1 minutes, then the lights were turned on and the mice were adapted for 2 minutes, and as soon as the mice were moved to the dark chamber, an electric shock was applied at 0.5mA for 1 second to conduct a learning test. A teat trial was conducted for each mouse on the day following the learning test. 5xFAD mice and 6xTg mice were placed in the chamber with lights on, and the time taken for all four paws of the 5xFAD mice and 6xTg mice to enter (latency time) was specified to within 300 seconds. As a result, as shown in FIG. 12, when the GTC310-01 antibody was administered to 5xFAD mice as well as 6xTg mice, the recovery was close to that of the normal control group.

**[Example 3] Identification of the epitope of the Lrig-1 protein**

To identify the epitope where the GTC310-01 antibody from the above preparation can bind in the extracellular domain of the Lrig-1 protein, the following experiments were performed.

**[3-1] Analytical samples, reagents, and materials**

**[3-1-1] Antigen (Antigen)**

Substance name: hTregL1-his
Component: Protein
Molecular weight: Approximately 83.9 kDa (excluding glycoprotein chains)
Storage conditions: -20°C
Test substance: hTregL1-his (0.5 mg/M$\ell$)

**[3-1-2] Antibody**

Substance name: GTC310-01-hlgG1
Component: Protein
Molecular weight: Approximately 159 kDa (excluding glycan chains)
Storage conditions: -20°C
Test Substance: GTC310-01-hlgG1 (2.7mg/M$\ell$)

**[3-1-3] Reagents and Materials**

CNBr-activated Sepharose 4B™ (GE, 17-0430-01)

Diethylpyrocarbonate (DEPC, Sigma, 159220)

Imidazole (Acros, 301870010)

Sequencing grade modified trypsin (Promega, V5117)

Chymotrypsin, Sequencing grade (Promega, V1062)

Disuccinimidyl dibutyric urea (DSBU; Thermo, A35459)

Dimethyl sulfoxide (DMSO) (Sigma, D5879)

Sodium bicarbonate (NaHCO$_3$) (Sigma, 792519)

Sodium chloride (NaCl, Sigma, S3014)

Sodium acetate (NaOAc, Sigma, S2889)

Tris (GE, 17-1321-01), Glycine (GE, 17-1323-01)

Acetone (Merck, 1.07021.2521)

Dithiothreitol (DTT; GE, 17-1318-02)

Iodoacetamide (IAA; Sigma, I-6125)

Sodium phosphate monobasic dihydrate ($NaH_2PO_4$, Sigma, 71505)

Sodium phosphate dibasic heptahydrate ($Na_2HPO_4$, Sigma, S9390)

Syringe filter (0.2 $\mu$m) (Sartorius Stedim, 16534)

Acetonitrile, water (HPLC grade, J.T. Baker)

Acetic acid (Sigma, 695092)

Formic acid (Sigma, 33015)

Urea (GE, 17-1319-01)

## [3-2] Experimental Methods

### [3-2-1] Covalent Labeling Mass Spectrometry (CL-MS)

The antigen and antibody ratio was adjusted to 2:1, and the antigen sample was prepared. DEPC was added to ensure the concentration did not exceed 1%, and the mixture was reacted at 37°C for 1 minute. Imidazole was then added to terminate the reaction. Subsequently, acetone was added to perform a precipitation reaction, followed by centrifugation to remove the supernatant. The precipitated protein was dissolved using urea.

The precipitated protein was then digested with trypsin and chymotrypsin, followed by removal of the sugar chains attached to the protein using PNGase-F. Finally, DTT and IAA were used to cleave the disulfide bonds between cysteine residues in the protein, followed by LC-MS, $MS^2$ analysis.

### [3-2-2] Cross-linking MS (XL-MS)

The antigen-to-antibody ratio was adjusted to 2:1, and two antigen samples were prepared. Cross-linker (DSBU) was dissolved in DMSO until fully dissolved. The sample and DSBU were mixed at a ratio of 1:100, and DMSO alone was added to the other sample. The mixture was reacted at 25°C for 1 hour. After the reaction was complete, the sample was digested using trypsin or chymotrypsin, followed by removal of glycan chains attached to the protein using PNGase-F. Finally, disulfide bonds between cysteine residues in the protein were cleaved using DTT and IAA, and LC-MS, $MS^2$ analysis was performed.

### [3-2-3] Peptide Screening

CNBr activation (cyanogen bromide activation) was performed. Specifically, 1 mM HCl was added to the CNBr resin. Then, the antibody was added and reacted at 25°C for 1 hour. Subsequently, washing was performed with coupling buffer (Coupling buffer: 0.1 M $NaHCO_3$, 0.5 M NaCl, pH 8.3) at 10 column volumes (CV). Next, 0.1 M Tris-HCl pH 8.0 was added at 5-10 CV, and the mixture was reacted at 25°C for 1 hour. After the reaction was complete, the column was washed with 5 column volumes of coupling buffer. Next, the column was washed with 0.1 M sodium acetate/acetic acid, 0.5 M NaCl, pH 4.0, for 5 column volumes. Subsequently, the column was washed with 0.1 M Tris-HCl, 0.5 M NaCl, pH 8.0, 5 column volumes, and the above acetic acid/acetic acid, Tris-HCl, NaCl washing process was repeated three times.

Immuno-affinity purification was then performed. Specifically, 50 $\mu$g of antigen was digested with trypsin and chymotrypsin, respectively, and the digested antigen was added to a CNBr-antibody column and reacted at room temperature for 1 hour. Then, the column was washed three times with 1X PBS at a volume of five column volumes. Subsequently, the elution buffer (0.1 M glycine, pH 2.4) was added at a volume of five column volumes, and the elution fractions were concentrated and analyzed by LC-MS and $MS^2$.

### [3-2-4] LC-MS, MS analysis conditions

#### 1. Liquid chromatography (LC) conditions

Column: ACQUITY UPLC CSH C18 (1.7 $\mu$m, 2.1 $\times$ 100 mm, Waters)

Flow rate: 100 μℓ/min

Mobile phase A: D.W / 0.1% formic acid

Mobile phase B: ACN / 0.1% formic acid

Temperature: 25 °C

LC conditions (gradient)

[Table 3]

| Step | Total time (min) | Flow rate (μℓ/min) | A (%) | B (%) |
|---|---|---|---|---|
| 0 | 0 | 100.0 | 98. | 2 |
| 1 | 5.0 | 100.0 | 98.0 | 2 |
| 8 | 5.10 | 100.0 | 95.0 | 5.0 |
| 3 | 50.0 | 100.0 | 70.0 | 30 |
| 4 | 51.00 | 100.0 | 5.0 | 95.0 |
| 5 | 61.00 | 100.00 | 5.0 | 95.0 |
| 6 | 62.00 | 100.0 | 98.00 | 2.0 |
| 7 | 71.00 | 100.00 | 98.00 | 2.0 |

## 2. MS, MS$^2$ Condition

[Table 4]

| HESI Source | |
|---|---|
| Sheath gas flow rate | 3 |
| Auxiliary gas flow rate | 10 |
| Sweep gas flow rate | 1 |
| Spray voltage (kV) | 3.5 |
| Spray current (μA) | - |
| Capillary temperature (°C) | 2 |
| S-lens RF level | 50 |
| Auxiliary gas heater temperature (°C) | 20 |

[Table 5]

| General | |
|---|---|
| Run time | 0 to 70 minutes |
| Polarity | Positive |
| Default Charge State | 2 |

[Table 6]

| Full MS | |
|---|---|
| Resolution | 7 |

(continued)

| Full MS | |
|---|---|
| AGC target | 3.00E+06 |
| Maximum IT | 100 ms |
| Scan range | 200 to 2,000 m/z |

[Table 7]

| dd-MS$^2$/ dd-SIM | |
|---|---|
| Resolution | 17,500 |
| AGC target | 1.00E+05 |
| Maximum IT | 100 ms |
| Loop count | 10 |
| TopN | 10 |
| Isolation window | 2.0 m/z |
| (N)CE / stepped (N)CE | 2 |

[Table 8]

| dd Settings | |
|---|---|
| Minimum AGC target | 8.00E+04 |
| Intensity threshold | 8.0E+05 |
| Charge exclusion | Unassigned, 7, 8, >8 |
| Peptide match | Preferred |
| Exclude isotopes | On |
| Dynamic exclusion | 10.0 |

## [3-3] Results

### [3-3-1] Covalent labeling MS (CL-MS)

Covalent labeling MS (CL-MS) is a method that modifies (labels) amino acid residues on the surface of proteins accessible to solvents, enabling the detection of protein structural changes through peptide mass fingerprinting and MS/MS analysis. Specifically, the test conditions involve labeling the antigen and the antigen-antibody complex separately and interpreting the MS analysis results to confirm changes in the antigen's surface structure (labeling ratio). Therefore, this method can also analyze structurally close epitopes that form conformational epitopes (non-discontinuous antigen-binding sites). In this study, DEPC was used to induce carboethoxy labeling (carboethoxy labeling; $COOC_2H_4$)on specific amino acid residues (H, Y, S, T, K, C, N-terminal of protein). The ratio of labeled peptides (modification; +72.021 Da) to non-labeled peptides (non-labeling peptide; normal) was investigated to identify peptides whose levels decrease in antigen-antibody (Ag+Ab, experimental group) complexes compared to antigen (Ag, control group) samples.

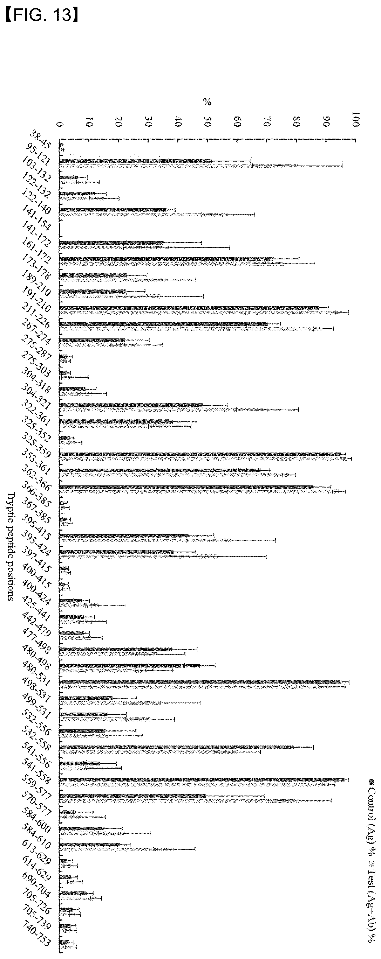

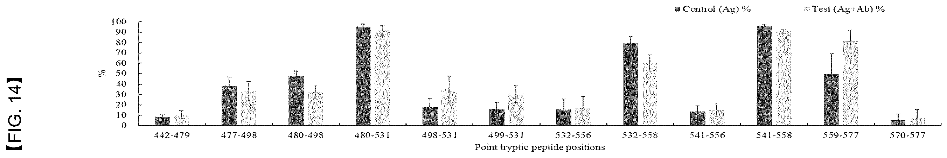

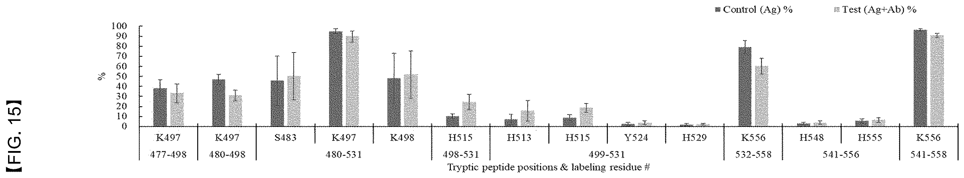

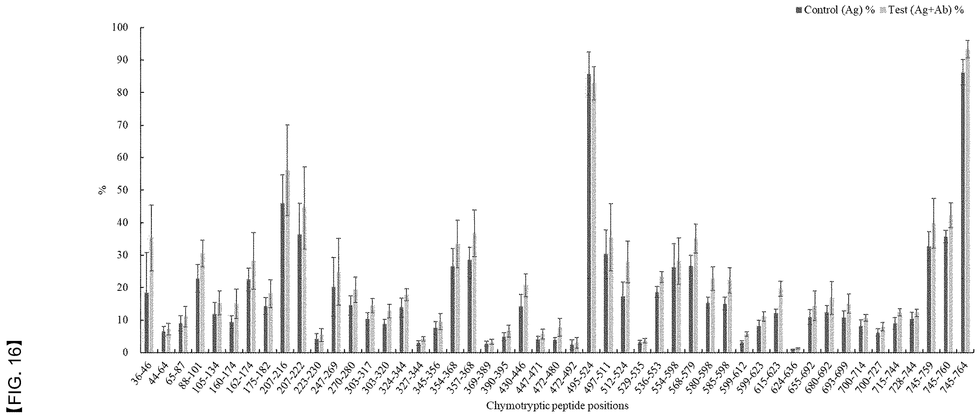

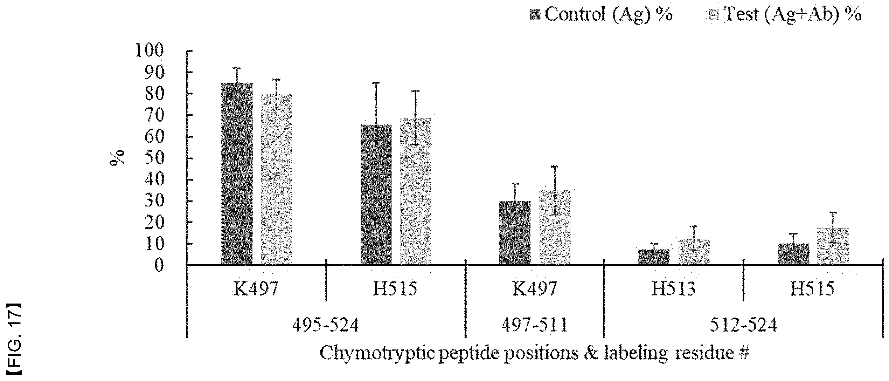

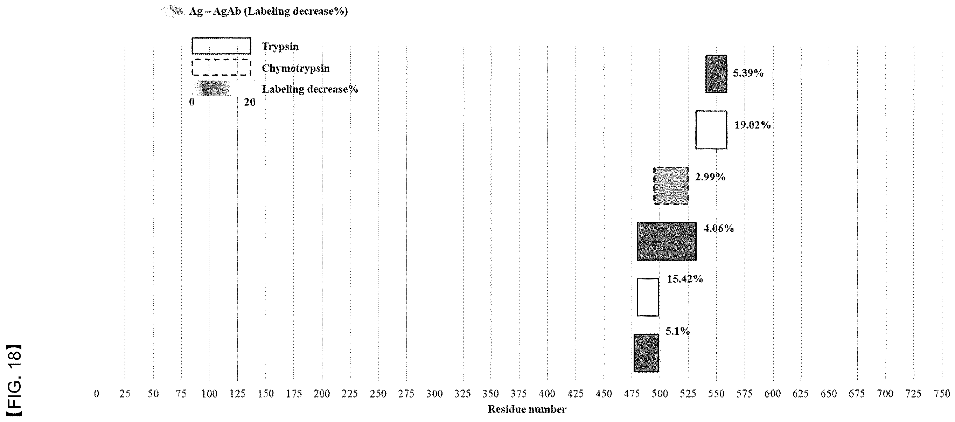

hTregL1-his (Ag, control group) and hTregL1-his/1C07-hIgG1 (Ag+Ab, experimental group) samples were prepared, and after DEPC treatment, the samples were digested with protease and analyzed in three replicate experiments. The MS analysis results of the control group (DEPC-labeled Ag) and the experimental group (DEPC-labeled Ag-Ab complex) were simultaneously matched with the hTregL1-his sequence using the BioPharma Finder program, and the labeled peptides were compared. Trypsin (T) or chymotrypsin (chymotrypsin; Y) were used to identify non-labeled and labeled peptides in the control and experimental group samples. The results of repeated experiments were summarized to compare the labeling ratios (percent, %) of the control and experimental groups based on amino acid sequence positions (Tables 9 and 12), and the results were plotted as bar graphs (FIG. 13, FIG. 16). The main intervals where a % decrease in DEPC labeling was observed were enlarged and re-plotted (Table 10, FIG. 14). To identify which amino acids contributed significantly to the decrease in DEPC labeling, the DEPC labeling decrease ratio was confirmed centered on the residues labeled by DEPC in the hydrolyzed fragments identified in the sequences where the decrease was observed (Table 11, 13, and FIGS. 15, 17). The results were applied to the hTregL1-his sequence (figure omitted). A histogram was created by plotting the degree of DEPC labeling reduction on the x-axis (0-20%) and the antigen sequence numbers on the y-axis, using a color spectrum (FIG. 18). Fragments showing a relatively higher reduction in DEPC labeling ratio from the blue to the red color spectrum were represented in red, while those showing a lower reduction were represented in the blue color spectrum. The regions with matching sequences from the two protease assay results used in this experiment were highlighted in red (figure omitted).

In trypsin, the sequences 477-498, 480-498, 480-531, 532-558, and 541-558 showed a DEPC labeling reduction of 4% or more (Table 11, FIG. 13). Additionally, lysine (lysine) residues at positions 497 and 556 showed a reduction rate ranging from a minimum of 5% to a maximum of 19% (Table 12, FIG. 14). In chymotrypsin, the sequence Y495-524 showed a approximately 3% reduction in DEPC labeling (Table 12, FIG. 16), and the DEPC labeling of lysine 495 exhibited a reduction rate of 5.12% compared to the control group (Table 13, FIG. 17).

Based on the CL-MS analysis results of hTregL1-his/GTC310-01-hlgG1, the sequence including lysine 497 is exposed externally, and the 495-524 sequence, which consistently showed a reduced DEPC labeling ratio in both trypsin and chymotrypsin experiments, is considered the most likely epitope.

[Table 9] Trypsin CL-MS labeling peptide%

| Position s | Control (Ag)% | | | Test (Ag+Ab)% | | | Labeling |
|---|---|---|---|---|---|---|---|
| | Mean | STEDV | RSD | Mean | STEDV | RSD | decrease % |
| 38 | 0.87 | 0.4 | 46.06 | 1.03 | 0.42 | 41.07 | -0.16 |
| 95-121 | 51.52 | 13.05 | 25.34 | 80.3 | 15.14 | 18.86 | -28.79 |
| 103-132 | 6.24 | 3.18 | 50.88 | 9.68 | 3.83 | 39.57 | -3.44 |
| 122-132 | 11.98 | 4.01 | 33.5 | 15.14 | *5.05* | 33.35 | -3.16 |
| 122-140 | 36.01 | 3.13 | 8.71 | 56.94 | 8.93 | 15.68 | -20.93 |
| 141-154 | 0.1 | 0.04 | 38.42 | 0.1 | 0.03 | 33.44 | 0 |
| 141-172 | 35.13 | 12.87 | 36.65 | 39.54 | 17.94 | 45.37 | -4.41 |
| 161-172 | 72.22 | 8.69 | 12.04 | 75.61 | 10.54 | 13.94 | -3.39 |
| 173-178 | 22.89 | 6.68 | 29.2 | 35.81 | 10.22 | 28.54 | -12.92 |
| 189-210 | 22.68 | 6.14 | 27.09 | 34.08 | 14.64 | 42.95 | -11.4 |
| 191-210 | 87.54 | 3.44 | 3.93 | 95.31 | 2.18 | 2.28 | -7.78 |
| 211-226 | 70.29 | 4.49 | 6.38 | 89.09 | 3.35 | 3.77 | -18.81 |
| 267-274 | 22.25 | 8.08 | 36.33 | 26.15 | 8.74 | 33.41 | -3.9 |
| 275-287 | 2.89 | 1.5 | 51.92 | 2.69 | 1.16 | 43.26 | 0.2 |
| 275-303 | 2.54 | 1.25 | 49.04 | 5.16 | 4.56 | 88.37 | -2.62 |
| 304-318 | 8.86 | 3.5 | 39.47 | 11.12 | 4.82 | 43.33 | -2.26 |
| 304-321 | 48.27 | 8.57 | 17.75 | 70.17 | 10.5 | 14.96 | -21.9 |
| 322-361 | 38.27 | 7.93 | 20.72 | 37.26 | 7.15 | 19.2 | 1.01 |
| 325-352 | 3.57 | 1.25 | 34.95 | 5.46 | 2.21 | 40.37 | -1.89 |
| 325-359 | 95 | 1.61 | 1.69 | 97.28 | 1.25 | 1.29 | -2.28 |
| 353-361 | 67.91 | 3.08 | 4.53 | 77.49 | 2.14 | 2.76 | -9.58 |
| 362-366 | 85.8 | 5.79 | 6.75 | 94.44 | 2.15 | 2.28 | -8.64 |
| 366-385 | 1.67 | 1.07 | 64.2 | 2.18 | 1.37 | 63 | -0.51 |
| 367-385 | 2.47 | 1.29 | 52.22 | 2.93 | 1.41 | 48.26 | -0.46 |
| 395-415 | 43.6 | 8.55 | 19.6 | 58.09 | 14.98 | 25.78 | -14.49 |
| 395-424 | 38.38 | 7.64 | 19.91 | 53.6 | 16.26 | 30.34 | -15.22 |
| 397-415 | 2.84 | 0.3 | 10.71 | 3.18 | 0.68 | 21.34 | -0.34 |

(continued)

| Position s | Control (Ag)% | | | Test (Ag+Ab)% | | | Labeling |
|---|---|---|---|---|---|---|---|
| | Mean | STEDV | RSD | Mean | STEDV | RSD | decrease % |
| 400-415 | 1.91 | 1.23 | 64.64 | 2.28 | 1.18 | 51.85 | -0.38 |
| 400-424 | 7.55 | 2.68 | 35.47 | 13.67 | 8.49 | 62.1 | -6.11 |
| 425-441 | 8.35 | 3.44 | 41.21 | 11.12 | 4.62 | 41.51 | -2.78 |
| 442-479 | 8.46 | 1.77 | 20.93 | 10.56 | 3.85 | 36.51 | -2.09 |
| **477-498** | **38.21** | **8.23** | **21.55** | **33.11** | **9.3** | **28.08** | **5.1** |
| **480-498** | **47.42** | **5.14** | **10.85** | **31.99** | **6.35** | **19.86** | **15.42** |
| **480-531** | **95.2** | **2.55** | **2.68** | **91.14** | **5.2** | **5.71** | **4.06** |
| 498-531 | 17.97 | 8.24 | 45.82 | 34.66 | 12.88 | 37.16 | -16.69 |
| 499-531 | 16.3 | 6.36 | 39.05 | 30.68 | 8.19 | 26.68 | -14.38 |
| 532-556 | 15.58 | 10.37 | 66.55 | 16.77 | 11.22 | 66.89 | -1.2 |
| **532-558** | **79.15** | **6.55** | **8.27** | **60.14** | **7.71** | **12.83** | **19.02** |
| 541-556 | 13.73 | 5.51 | 40.14 | 14.99 | 6.03 | 40.2 | -1.27 |
| **541-558** | **96.33** | **1.29** | **1.34** | **90.94** | **2.07** | **2.27** | **5.39** |
| 559-577 | 49.34 | 19.8 | 40.13 | 81.28 | 10.56 | 12.99 | -31.95 |
| 570-577 | 5.43 | 5.9 | 108.65 | 7.34 | 8.12 | 110.63 | -1.91 |
| 584-600 | 15.17 | 6.04 | 39.83 | 22 | 8.6 | 39.11 | -6.83 |
| 584-610 | 20.52 | 3.45 | 16.79 | 38.81 | 7.03 | 18.11 | -18.29 |
| 613-629 | 2.64 | 1.76 | 66.59 | 3.75 | 2.39 | 63.59 | -1.11 |
| 614-629 | 4.07 | 2.02 | 49.52 | 5.2 | 2.51 | 48.23 | -1.13 |
| 690-704 | 9.27 | 2.19 | 23.58 | 12.4 | 1.84 | 14.86 | -3.13 |
| 705-726 | 4.66 | 2.03 | 43.48 | 5.32 | 1.86 | 35 | -0.66 |
| 705-739 | 3.75 | 1.79 | 47.84 | 3.88 | 1.92 | 49.57 | -0.13 |
| 740-753 | 3.15 | 1.71 | 54.25 | 3.9 | 1.85 | 47.34 | -0.75 |

[Table 10] Trypsin CL-MS labeling peptide % (point tryptic peptide positions)

| Position s | Control (Ag) % | | | Test (Ag+Ab) % | | | Labeling |
|---|---|---|---|---|---|---|---|
| | Mean | STEDV | RSD | Mean | STEDV | RSD | decrease % |
| 442-479 | 8.4 | 1.77 | 20.93 | 10.56 | 3.85 | 36.51 | -2.09 |
| **477-498** | **38.21** | **8.23** | **21.55** | **33.11** | **9.3** | **28.08** | **5.1** |
| **480-498** | **47.42** | **5.14** | **10.85** | **31.99** | **6.35** | **19.86** | **15.42** |
| **480-531** | **95.2** | **2.55** | **2.68** | **91.14** | **5.2** | **5.71** | **4.06** |
| 498-531 | 17.97 | 8.24 | 45.82 | 34.66 | 12.88 | 37.16 | -16.69 |
| 499-531 | 16.3 | 6.36 | 39.05 | 30.68 | 8.19 | 26.68 | -14.38 |
| 532-556 | 15.58 | 10.37 | 66.55 | 16.77 | 11.22 | 66.89 | -1.2 |
| **532-558** | **79.15** | **6.55** | **8.27** | **60.14** | **7.71** | **12.83** | **19.02** |
| 541-556 | 13.73 | 5.51 | 40.14 | 14.99 | 6.03 | 40.2 | -1.27 |
| **541-558** | **96.33** | **1.29** | **1.34** | **90.94** | **2.07** | **2.27** | **5.39** |
| 559-577 | 49.34 | 19.8 | 40.13 | 81.28 | 10.56 | 12.99 | -31.95 |

(continued)

| Position s | Control (Ag) % | | | Test (Ag+Ab) % | | | Labeling |
|---|---|---|---|---|---|---|---|
| | Mean | STEDV | RSD | Mean | STEDV | RSD | decrease % |
| 570-577 | 5.43 | 5.9 | 108.65 | 7.34 | 8.12 | 110.63 | -1.91 |

[Table 11] Trypsin CL-MS labeling peptide% (tryptic peptide positions & labeling residue)

| Positio ns | Residue # | Control (Ag)% | | | Test (Ag+Ab)% | | | Labelin g |
|---|---|---|---|---|---|---|---|---|
| | | Mean | STEDV | RSD | Mean | STEDV | RSD | decreas e% |
| 477 | **K497** | **38.2** | **8.23** | **21.55** | **33.11** | **9.3** | **28.08** | **5.1** |
| 480-498 | **K497** | **47.09** | **4.92** | **10.45** | **30.82** | **5.33** | **17.31** | **16.27** |
| 480-531 | S483 | 45.77 | 24.61 | 53.76 | 50.33 | 23.53 | 46.74 | -4.56 |
| | **K497** | **94.88** | **2.56** | **2.7** | **89.64** | **5.61** | **6.26** | **5.24** |
| | K498 | 48.13 | 24.86 | 51.65 | 51.81 | 23.66 | 45.66 | -3.68 |
| 498-531 | H515 | 10.27 | 2.38 | 23.13 | 24.33 | 7.63 | 31.36 | -14.06 |
| 499-531 | H513 | 7.08 | 5 | 70.62 | 15.71 | 10.3 | 65.59 | -8.63 |
| | H515 | 8.68 | 3.13 | 36.04 | 18.56 | 4.27 | 23.02 | -9.88 |
| | Y524 | 2.54 | 1.63 | 64.19 | 3.92 | 1.92 | 48.89 | -1.39 |
| | H529 | 1.83 | 1.01 | 55.12 | 2.35 | 0.81 | 34.42 | -0.52 |
| 532-558 | **K556** | **79.15** | **6.55** | **8.27** | **60.14** | **7.71** | **12.83** | **19.02** |
| 541-556 | H548 | 2.94 | 1.21 | 41.05 | 3.94 | 1.76 | 44.71 | -0.99 |
| | H555 | 5.65 | 2.07 | 36.61 | 6.3 | 2.31 | 36.57 | -0.65 |
| 541-558 | **K556** | **96.33** | **1.29** | **1.34** | **90.94** | **2.07** | **2.27** | **5.39** |

[Table 12] Chymotrypsin CL-MS labeling peptide%

| Position s | Control (Ag)% | | | Test (Ag+Ab)% | | | Labeling |
|---|---|---|---|---|---|---|---|
| | Mean | STEDV | RSD | Mean | STEDV | RSD | decrease % |
| 36 | 18.39 | 12.45 | 67.71 | 35.31 | 10.1 | 28.62 | -16.92 |
| 44-64 | 6.48 | 1.48 | 22.78 | 7.21 | 1.74 | 24.12 | -0.73 |
| 65-87 | 8.99 | 2.28 | 25.39 | 11.05 | 3.13 | 28.33 | -2.06 |
| 88-101 | 22.83 | 4.26 | 18.68 | 30.48 | 4.11 | 13.47 | -7.65 |
| 105-134 | 11.89 | 3.54 | 29.77 | 15.25 | 3.63 | 23.83 | -3.36 |
| 160-174 | 9.33 | 1.99 | 21.28 | 14.93 | 4.52 | 30.25 | -5.59 |
| 162-174 | 22.46 | 3.51 | 15.61 | 28.19 | 8.7 | 30.85 | -5.73 |
| 175-182 | 14.31 | 2.56 | 17.88 | 18.05 | 4.27 | 23.67 | -3.73 |
| 207-216 | 45.97 | 8.71 | 18.94 | 56.06 | 13.99 | 24.96 | -10.08 |
| 207-222 | 36.46 | 9.5 | 26.06 | 44.53 | 12.65 | 28.41 | -8.07 |
| 223-230 | 4.27 | 1.58 | 36.93 | 5.38 | 1.96 | 36.42 | -1.11 |
| 247-269 | 20.22 | 9.06 | 44.82 | 24.84 | 10.31 | 41.52 | -4.63 |
| 270-280 | 14.52 | 2.94 | 20.26 | 19.33 | 3.96 | 20.49 | -4.82 |
| 303-317 | 10.32 | 1.91 | 18.49 | 14.46 | 2.2 | 15.24 | -4.14 |
| 303-320 | 8.79 | 1.43 | 16.24 | 12.75 | 2.08 | 16.28 | -3.96 |
| 324-344 | 13.84 | 2.95 | 21.31 | 17.77 | 1.9 | 10.69 | -3.93 |

(continued)

| Position s | Control (Ag)% | | | Test (Ag+Ab)% | | | Labeling |
|---|---|---|---|---|---|---|---|
| | Mean | STEDV | RSD | Mean | STEDV | RSD | decrease % |
| 327-344 | 2.91 | 0.74 | 25.43 | 4.2 | 0.72 | 17.08 | -1.28 |
| 345-356 | 7.59 | 1.94 | 25.52 | 9.56 | 2.47 | 25.83 | -1.97 |
| 354-368 | 26.56 | 5.51 | 20.73 | 33.5 | 7.28 | 21.75 | -6.94 |
| 357-368 | 28.62 | 3.81 | 13.33 | 36.73 | 7.16 | 19.49 | -8.1 |
| 369-389 | 2.75 | 0.71 | 25.99 | 3.31 | 0.8 | 24.17 | -0.56 |
| 390-395 | 4.87 | 1.2 | 24.6 | 6.61 | 1.82 | 27.45 | -1.75 |
| 430-446 | 14.14 | 3.68 | 26.01 | 20.74 | 3.54 | 17.09 | -6.6 |
| 447-471 | 4.07 | 0.94 | 23.15 | 5.76 | 1.49 | 25.91 | -1.68 |
| 472-480 | 3.89 | 0.78 | 20.02 | 7.77 | 2.75 | 35.44 | -3.88 |
| 472-492 | 2.39 | 1.49 | 62.11 | 2.97 | 1.7 | 57.22 | -0.58 |
| **495-524** | **85.83** | **6.65** | **7.75** | **82.83** | **5.16** | **6.23** | **2.99** |
| 497-511 | 30.35 | 7.36 | 24.24 | 35.5 | 10.28 | 28.96 | -5.15 |
| 512-524 | 17.28 | 4.4 | 25.46 | 27.87 | 6.49 | 23.29 | -10.59 |
| 529-535 | 3.14 | 0.69 | 21.85 | 3.7 | 0.7 | 18.98 | -0.56 |
| 536-553 | 18.59 | 1.67 | 8.97 | 23.25 | 1.7 | 7.32 | -4.67 |
| 554-598 | 26.27 | 7.25 | 27.58 | 28.14 | 7.15 | 25.4 | -1.87 |
| 568-579 | 26.77 | 3.2 | 11.95 | 35.05 | 4.42 | 12.62 | -8.28 |
| 580-598 | 15.27 | 1.7 | 11.13 | 22.76 | 3.66 | 16.08 | -7.49 |
| 585-598 | 15.04 | 1.97 | 13.1 | 22.24 | 3.98 | 17.88 | -7.2 |
| 599-612 | 3.07 | 0.64 | 20.88 | 5.74 | 0.67 | 11.7 | -2.67 |
| 599-623 | 8.19 | 1.75 | 21.33 | 11.1 | 1.4 | 12.58 | -2.91 |
| 615-623 | 12.11 | 1.22 | 10.08 | 19.61 | 2.3 | 11.73 | -7.5 |
| 624-636 | 0.92 | 0.13 | 14.13 | 1.26 | 0.2 | 16.16 | -0.34 |
| 655-692 | 10.91 | 2.24 | 20.57 | 14.35 | 4.56 | 31.8 | -3.44 |
| 680-692 | 12.33 | 2.14 | 17.37 | 16.76 | 5.06 | 30.16 | -4.43 |
| 693-699 | 10.82 | 2.02 | 18.65 | 15.01 | 2.94 | 19.58 | -4.19 |
| 700-714 | 8.1 | 1.95 | 24.06 | 10.66 | 1 | 9.42 | -2.55 |
| 700-727 | 6.14 | 1.18 | 19.23 | 7.93 | 1.3 | 16.35 | -1.79 |
| 715-744 | 8.96 | 1.78 | 19.88 | 12.39 | 1.08 | 8.7 | -3.43 |
| 728-744 | 10.4 | 2.03 | 19.49 | 12.18 | 1.2 | 9.84 | -1.78 |
| 745-759 | 32.69 | 4.47 | 13.68 | 39.84 | 7.65 | 19.21 | -7.14 |
| 745-760 | 35.66 | 1.89 | 5.31 | 42.25 | 3.88 | 9.19 | -6.59 |
| 745-764 | 86.24 | 3.94 | 4.57 | 93.32 | 2.64 | 2.83 | -7.08 |

[Table 13] Chymotrypsin CL-MS labeling peptide% (chymotryptic peptide positions & labeling residue)

| Positio ns | Residue # | Control (Ag)% | | | Test (Ag+Ab)% | | | Labelin g |
|---|---|---|---|---|---|---|---|---|
| | | Mean | STEDV | RSD | Mean | STEDV | RSD | decreas e% |
| 495-524 | **K497** | **84.9** | **6.97** | **8.21** | **79.78** | **6.79** | **8.52** | **5.12** |

(continued)

| Positio ns | Residue # | Control (Ag)% | | | Test (Ag+Ab)% | | | Labelin g |
|---|---|---|---|---|---|---|---|---|
| | | Mean | STEDV | RSD | Mean | STEDV | RSD | decreas e% |
| | H515 | 65.56 | 19.61 | 29.91 | 68.69 | 12.43 | 18.09 | -3.13 |
| 497-511 | K497 | 30.1 | 7.78 | 25.83 | 34.83 | 11.29 | 32.41 | -4.73 |
| 512-524 | H513 | 7.53 | 2.66 | 35.28 | 12.47 | 5.52 | 44.25 | -4.94 |
| | H515 | 10.12 | 4.52 | 44.67 | 17.43 | 7.11 | 40.81 | -7.3 |

[Table 14] CL-MS analysis labeling peptide% (epitope candidate)

| Enzym e | Position s | Control (Ag)% | | | Test (Ag+Ab)% | | | Labelin g |
|---|---|---|---|---|---|---|---|---|
| | | Mean | STEDV | RSD | Mean | STEDV | RSD | decreas e% |
| T | 477-498 | 38.2 | 8.23 | 21.55 | 33.11 | 9.3 | 28.08 | 5.1 |
| T | 480-498 | 47.42 | 5.14 | 10.85 | 31.99 | 6.35 | 19.86 | 15.42 |
| T | 480-531 | 95.2 | 2.55 | 2.68 | 91.14 | 5.2 | 5.71 | 4.06 |
| Y | 495-524 | 85.83 | 6.65 | 7.75 | 82.83 | 5.16 | 6.23 | 2.99 |
| T | 532-558 | 79.15 | 6.55 | 8.27 | 60.14 | 7.71 | 12.83 | 19.02 |
| T | 541-558 | 96.33 | 1.29 | 1.34 | 90.94 | 2.07 | 2.27 | 5.39 |

## [3-3-2] Cross-linking MS (XL-MS)

The analytical method combining chemical cross-linking and mass spectrometry (MS) is used not only for protein-protein interactions and epitope mapping analysis but also for analyzing complexes between peptides, DNA, RNA, and proteins. This test was conducted using a chemical cross-linker (Disuccinimidyl dibutyric urea; DSBU) that binds to the primary amine of proteins. It generally binds best to the N-terminus and lysine (K) of proteins and is known to react with serine (S) and threonine (Y) in experiments. The peptide-linker binding forms identified in MS analysis are widely known to experts as mono-link, loop-link, and cross-link. The cross-link analysis strategy in the epitope mapping (EP) test compares the control group (without cross-linker) and the experimental group (with cross-linker) samples to detect whether the antigen EP site and antibody are connected by the linker and detected or reduced compared to the control group. without crosslinker) and experimental group (test; with cross-linker) samples to identify fragments (peptides) that are not detected or reduced by less than 10% in the experimental group compared to the control group, indicating that the antigen EP site and antibody are linked by the linker. Cases where only the linker is bound to the peptide were calculated as XL% to further investigate that the site is not an EP site.

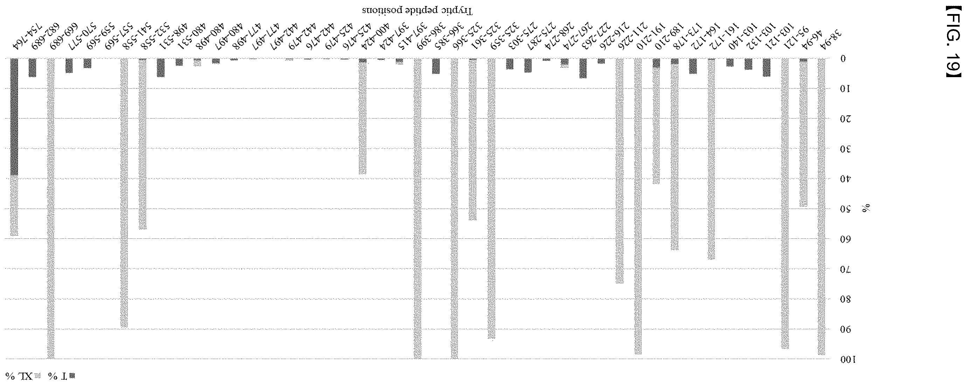

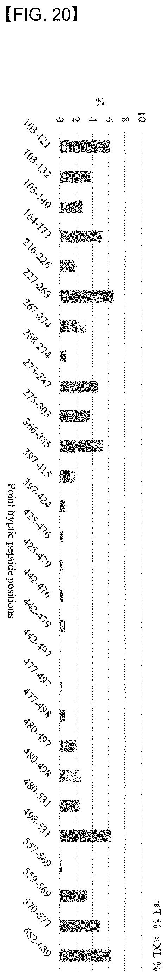

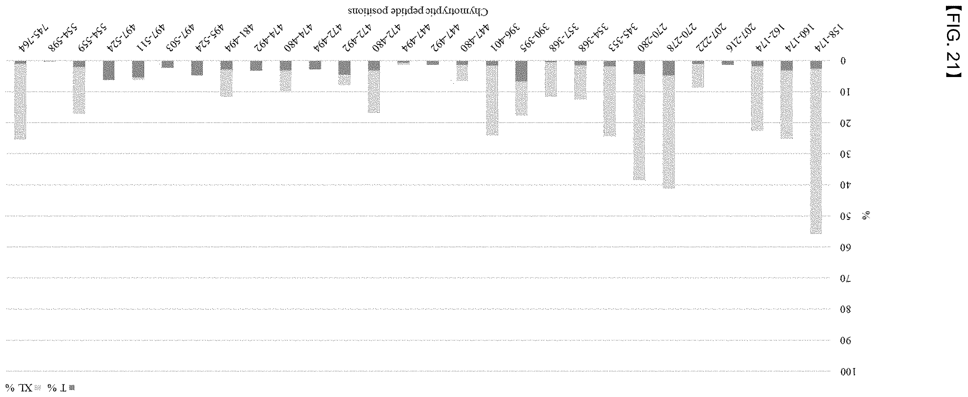

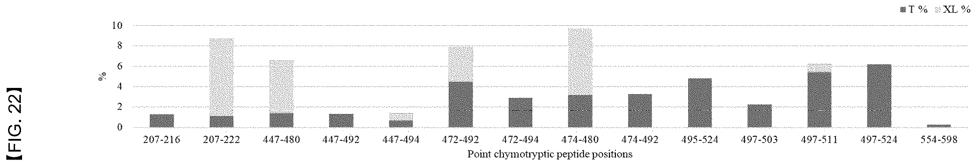

Prepare hTregL1-his/1C07-hIgG1 mixture samples, treat only the experimental group samples with a crosslinker (DSBU), and then digest with a protease. Peptides detected at 10% (Test%; T%) or less in the experimental group compared to the control group were summarized (Tables 15 and 17). We reviewed the sequences of peptides in the experimental group samples that met the condition of Test+XL% being 10% or less, including those with modifications where only the linker was bound (XL%) (Tables 16, 18, and FIGS. 19-22).

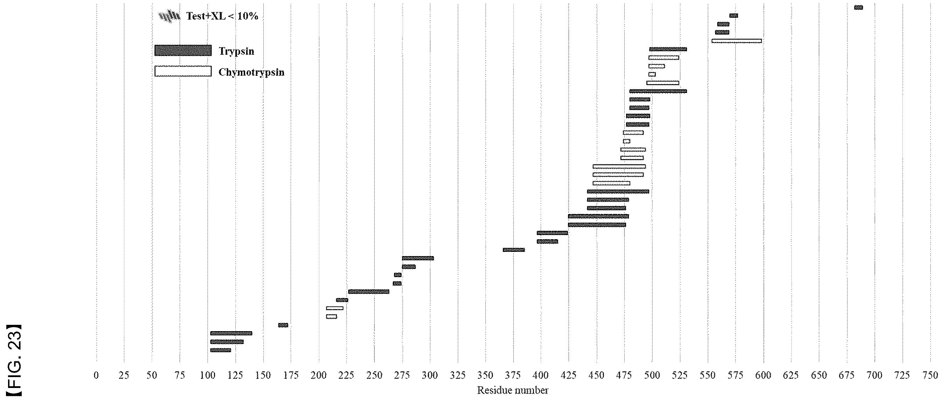

In the hTregL1-his sequence, three regions (216-222, 447-524, 557-577) showed a reduced ratio (T%) of peptides compared to the control group, with values of 10% or less (Table 19, FIG. 23). Based on the residues where the cross-linker binds, the K415 and K566 regions are interpreted as peptide reductions caused by cross-linker binding alone, while the K460, 476, 497, and 498 regions still show a reduction rate of 10% or less. The epitope site identified through XL-MS analysis was confirmed to be the 425-531 region. Among the sequences recognized by both trypsin and chymotrypsin at 10% or less, the remaining sequences excluding 425-531 were judged to be non-specific cross-linked.

[Table 15] Trypsin XL-MS analysis (detected peptides)

| Positions | Control% | Test% | XL% | Test + XL% | Linker site |
|---|---|---|---|---|---|
| 38-94 | 1.45 | 0.01 | 98.5 | 98.55 | S42, Y43, K45, K92 |
| 46-94 | 50.6 | 1.12 | 48.27 | 49.4 | K92 |
| 95-121 | 3.45 | 0.04 | 96.51 | 96.55 | K102 |

(continued)

| Positions | Control% | Test% | XL% | Test + XL% | Linker site |
|-----------|----------|-------|-----|------------|-------------|
| **103-121** | **93.82** | **6.18** | **0** | **6.18** | |
| **103-132** | **96.17** | **3.83** | **0** | **3.83** | |
| **103-140** | **97.22** | **2.78** | **0** | **2.78** | |
| 161-172 | 33.09 | 0.55 | 66.35 | 66.91 | K163 |
| **164-172** | **94.78** | **5.22** | **0** | **5.22** | |
| 173-178 | 36.32 | 1.93 | 61.75 | 63.68 | K175 |
| 189-210 | 58.16 | 3.11 | 38.74 | 41.84 | K207 |
| 191-210 | 1.5 | 0.14 | 98.37 | 98.5 | S202, K207 |
| 211-226 | 25.1 | 0.01 | 74.87 | 74.89 | K215 |
| **216-226** | **98.2** | **1.8** | **0** | **1.8** | |
| **227-263** | **93.33** | **6.67** | **0** | **6.67** | |
| **267-274** | **96.82** | **2.1** | **1.08** | **3.18** | **K267** |
| **268-274** | **99.21** | **0.79** | **0** | **0.79** | |
| **275-287** | **95.25** | **4.75** | **0** | **4.75** | |
| **275-303** | **96.32** | **3.68** | **0** | **3.68** | |
| 325-359 | 6.87 | 0.12 | 93 | 93.13 | K352 |
| 325-361 | 46.22 | 0.56 | 53.22 | 53.78 | K352, K359 |
| 325-366 | 0 | 0 | 100 | 100 | K352, K359, K361, K365 |
| **366-385** | **94.77** | **5.23** | **0** | **5.23** | |
| 386-399 | 0 | 0 | 100 | 100 | K394, K396 |
| **397-415** | **98.01** | **1.25** | **0.74** | **1.99** | **K399** |
| **397-424** | **99.39** | **0.61** | **0** | **0.61** | |
| 400-424 | 61.5 | 1.35 | 37.14 | 38.5 | K415 |
| **425-476** | **99.58** | **0.42** | **0** | **0.42** | |
| **425-479** | **99.68** | **0.32** | **0** | **0.32** | |
| **442-476** | **99.6** | **0.4** | **0** | **0** | |
| **442-479** | **99.37** | **0.28** | **0.35** | **0.63** | **K460, K476** |
| **442-497** | **99.9** | **0.1** | **0** | **0** | |
| **477-497** | **99.77** | **0.23** | **0** | **0.23** | |
| **477-498** | **99.31** | **0.69** | **0** | **0.69** | |
| **480-497** | **98.05** | **1.62** | **0.33** | **1.95** | **S490** |
| **480-498** | **97.4** | **0.68** | **1.92** | **2.6** | **S488, K497, K498** |
| **480-531** | **97.58** | **2.42** | **0** | **2.42** | |
| **498-531** | **93.73** | **6.27** | **0** | **6.27** | |
| 532-558 | 43.09 | 0.61 | 56.3 | 56.91 | K556 |
| 541-558 | 10.44 | 0.19 | 89.37 | 89.56 | K556 |
| **557-569** | **99.81** | **0.19** | **0** | **0.19** | |
| **559-569** | **96.67** | **3.33** | **0** | **3.33** | |
| **570-577** | **95.08** | **4.92** | **0** | **4.92** | |

(continued)

| Positions | Control% | Test% | XL% | Test + XL% | Linker site |
|---|---|---|---|---|---|
| 669-689 | 0 | 0 | 100 | 100 | K681 |
| **682-689** | **93.78** | **6.22** | **0** | **6.22** | |
| 754-764 | 41.05 | 38.96 | 20 | 58.95 | K754, T758 |

[Table 16] Trypsin XL-MS (decreased peptides)

| Positions | Control% | Test% | XL% | Test + XL% | Linker site |
|---|---|---|---|---|---|
| 103-121 | 93.8 | 6.18 | 0 | 6.18 | |
| 103-132 | 96.17 | 3.83 | 0 | 3.83 | |
| 103-140 | 97.22 | 2.78 | 0 | 2.78 | |
| 164-172 | 94.78 | 5.22 | 0 | 5.22 | |
| 216-226 | 98.2 | 1.8 | 0 | 1.8 | |
| 227-263 | 93.33 | 6.67 | 0 | 6.67 | |
| 267-274 | 96.82 | 2.1 | 1.08 | 3.18 | K267 |
| 268-274 | 99.21 | 0.79 | 0 | 0.79 | |
| 275-287 | 95.25 | 4.75 | 0 | 4.75 | |
| 275-303 | 96.32 | 3.68 | 0 | 3.68 | |
| 366-385 | 94.77 | 5.23 | 0 | 5.23 | |
| 397-415 | 98.01 | 1.25 | 0.74 | 1.99 | K399 |
| 397-424 | 99.39 | 0.61 | 0 | 0.61 | |
| 425-476 | 99.58 | 0.42 | 0 | 0.42 | |
| 425-479 | 99.68 | 0.32 | 0 | 0.32 | |
| 442-476 | 99.6 | 0.4 | 0 | 0.4 | |
| 442-479 | 99.37 | 0.28 | 0.35 | 0.63 | K460, K476 |
| 442-497 | 99.9 | 0.1 | 0 | 0 | |
| 477-497 | 99.77 | 0.23 | 0 | 0.23 | |
| 477-498 | 99.31 | 0.69 | 0 | 0.69 | |
| 480-497 | 98.05 | 1.62 | 0.33 | 1.95 | S490 |
| 480-498 | 97.4 | 0.68 | 1.92 | 2.6 | S488, K497, K498 |
| 480-531 | 97.58 | 2.42 | 0 | 2.42 | |
| 498-531 | 93.73 | 6.27 | 0 | 6.27 | |
| 557-569 | 99.81 | 0.19 | 0 | 0.19 | |
| 559-569 | 96.67 | 3.33 | 0 | 3.33 | |
| 570-577 | 95.08 | 4.92 | 0 | 4.92 | |
| 682-689 | 93.78 | 6.22 | 0 | 6.22 | |

[Table 17] Chymotrypsin XL-MS (detected peptides)

| Positions | Control% | Test% | XL% | Test + XL% | Linker site |
|---|---|---|---|---|---|
| 158-174 | 44.2 | 2.54 | 53.2 | 55.7 | K163 |
| 160-174 | 74.72 | 3.13 | 22.15 | 25.28 | K163 |
| 162-174 | 77.47 | 1.73 | 20.79 | 22.53 | K163 |

(continued)

| Positions | Control% | Test% | XL% | Test + XL% | Linker site |
|---|---|---|---|---|---|
| 207-216 | 98.69 | 1.31 | 0 | 1.31 | |
| 207-222 | 91.31 | 1.15 | 7.55 | 8.69 | K207, K215 |
| 270-278 | 58.96 | 4.78 | 36.27 | 41.04 | K274 |
| 270-280 | 61.67 | 4.41 | 33.93 | 38.33 | K274 |
| 345-353 | 75.54 | 1.93 | 22.52 | 24.46 | K352 |
| 354-368 | 87.51 | 1.51 | 10.97 | 12.49 | K359, K361, K365 |
| 357-368 | 88.42 | 0.58 | 11 | 11.58 | K359, K361, K365 |
| 390-395 | 82.37 | 6.63 | 11 | 17.63 | K394 |
| 396-401 | 75.89 | 1.66 | 22.45 | 24.11 | K396, K399 |
| 447-480 | 93.41 | 1.44 | 5.15 | 6.59 | K460, K476 |
| 447-492 | 98.67 | 1.33 | 0 | 1.33 | |
| 447-494 | 98.58 | 0.71 | 0.71 | 1.42 | S490 |
| 472-480 | 83.13 | 3.1 | 13.77 | 16.87 | K476 |
| 472-492 | 92.07 | 4.48 | 3.45 | 7.93 | K476 |
| 472-494 | 97.11 | 2.89 | 0 | 2.89 | |
| 474-480 | 90.29 | 3.17 | 6.54 | 9.71 | K476 |
| 474-492 | 96.74 | 3.26 | 0 | 3.26 | |
| 481-494 | 88.47 | 2.83 | 8.71 | 11.53 | S487 |
| 495-524 | 95.2 | 4.8 | 0 | 4.8 | |
| 497-503 | 97.74 | 2.26 | 0 | 2.26 | |
| 497-511 | 93.76 | 5.44 | 0.8 | 6.24 | K497, K498 |
| 497-524 | 93.79 | 6.21 | 0 | 6.21 | |
| 554-559 | 82.86 | 2.09 | 15.04 | 17.14 | K556 |
| 554-598 | 99.71 | 0.29 | 0 | 0.29 | |
| 745-764 | 74.58 | 0.98 | 24.44 | 25.42 | K754, T758 |

[Table 18] Chymotrypsin XL-MS (decreased peptides)

| Positions | Control% | Test% | XL% | Test + XL% | Linker site |
|---|---|---|---|---|---|
| 207-216 | 98.6 | 1.31 | 0 | 1.31 | |
| 207-222 | 91.31 | 1.15 | 7.55 | 8.69 | K207, K215 |
| 447-480 | 93.41 | 1.44 | 5.15 | 6.59 | K460, K476 |
| 447-492 | 98.67 | 1.33 | 0 | 1.33 | |
| 447-494 | 98.58 | 0.71 | 0.71 | 1.42 | S490 |
| 472-492 | 92.07 | 4.48 | 3.45 | 7.93 | K476 |
| 472-494 | 97.11 | 2.89 | 0 | 2.89 | |
| 474-480 | 90.29 | 3.17 | 6.54 | 9.71 | K476 |
| 474-492 | 96.74 | 3.26 | 0 | 3.26 | |
| 495-524 | 95.2 | 4.8 | 0 | 4.8 | |
| 497-503 | 97.74 | 2.26 | 0 | 2.26 | |

(continued)

| Positions | Control% | Test% | XL% | Test + XL% | Linker site |
|---|---|---|---|---|---|
| 497-511 | 93.76 | 5.44 | 0.8 | 6.24 | K497, K498 |
| 497-524 | 93.79 | 6.21 | 0 | 6.21 | |
| 554-598 | 99.71 | 0.29 | 0 | 0.29 | |

[Table 19] XL-MS Comprehensive Results

| Enzyme | Positions | Control% | Test% | XL% | Test + XL% | Linker site |
|---|---|---|---|---|---|---|
| T | 103-121 | 93.8 | 6.1 | 0 | 6.18 | |
| T | 103-132 | 96.17 | 3.83 | 0 | 3.83 | |
| T | 103-140 | 97.22 | 2.78 | 0 | 2.78 | |
| T | 164-172 | 94.78 | 5.22 | 0 | 5.22 | |
| Y | 207-216 | 98.69 | 1.31 | 0 | 1.31 | |
| Y | 207-222 | 91.31 | 1.15 | 7.55 | 8.69 | K207, K215 |
| T | 216-226 | 98.2 | 1.8 | 0 | 1.8 | |
| T | 227-263 | 93.33 | 6.67 | 0 | 6.67 | |
| T | 267-274 | 96.82 | 2.1 | 1.08 | 3.18 | K267 |
| T | 268-274 | 99.21 | 0.79 | 0 | 0.79 | |
| T | 275-287 | 95.25 | 4.75 | 0 | 4.75 | |
| T | 275-303 | 96.32 | 3.68 | 0 | 3.68 | |
| T | 366-385 | 94.77 | 5.23 | 0 | 5.23 | |
| T | 397-415 | 98.01 | 1.25 | 0.74 | 1.99 | K399 |
| T | 397-424 | 99.39 | 0.61 | 0 | 0.61 | |
| T | 425-476 | 99.58 | 0.42 | 0 | 0.42 | |
| T | 425-479 | 99.68 | 0.32 | 0 | 0.32 | |
| T | 442-476 | 99.6 | 0.4 | 0 | 0.4 | |
| T | 442-479 | 99.37 | 0.28 | 0.35 | 0.63 | K460, K476 |
| T | 442-497 | 99.9 | 0.1 | 0 | 0.1 | |
| Y | 447-480 | 93.41 | 1.44 | 5.15 | 6.59 | K460, K476 |
| Y | 447-492 | 98.67 | 1.33 | 0 | 1.33 | |
| Y | 447-494 | 98.58 | 0.71 | 0.71 | 1.42 | S490 |
| Y | 472-492 | 92.07 | 4.48 | 3.45 | 7.93 | K476 |
| Y | 472-494 | 97.11 | 2.89 | 0 | 2.89 | |
| Y | 474-480 | 90.29 | 3.17 | 6.54 | 9.71 | K476 |
| Y | 474-492 | 96.74 | 3.26 | 0 | 3.26 | |
| T | 477-497 | 99.77 | 0.23 | 0 | 0.23 | |
| T | 477-498 | 99.31 | 0.69 | 0 | 0.69 | |
| T | 480-497 | 98.05 | 1.62 | 0.33 | 1.95 | S490 |
| T | 480-498 | 97.4 | 0.68 | 1.92 | 2.6 | S488, K497, K498 |
| T | 480-531 | 97.58 | 2.42 | 0 | 2.42 | |
| Y | 495-524 | 95.2 | 4.8 | 0 | 4.8 | |

(continued)

| Enzyme | Positions | Control% | Test% | XL% | Test + XL% | Linker site |
|--------|-----------|----------|-------|-----|------------|-------------|
| Y | 497-503 | 97.74 | 2.26 | 0 | 2.26 | |
| Y | 497-511 | 93.76 | 5.44 | 0.8 | 6.24 | K497, K498 |
| Y | 497-524 | 93.79 | 6.21 | 0 | 6.21 | |
| T | 498-531 | 93.73 | 6.27 | 0 | 6.27 | |
| Y | 554-598 | 99.71 | 0.29 | 0 | 0.29 | |
| T | 557-569 | 99.81 | 0.19 | 0 | 0.19 | |
| T | 559-569 | 96.67 | 3.33 | 0 | 3.33 | |
| T | 570-577 | 95.08 | 4.92 | 0 | 4.92 | |
| T | 682-689 | 93.78 | 6.22 | 0 | 6.22 | |

**[3-3-3] Peptide Screening (Affinity-MS)**

The most common epitope mapping analysis method using a mass spectrometer (MS) is "epitope excision" or "epitope extraction." This test applied the epitope extraction method. A mixture of hTregL1-his antigen (Ag) peptides generated by proteolytic degradation (trypsin, chymotrypsin) is reacted with the prepared GTC310-01-hIgG1 antibody (Ab) and CNBr sepharose (cyanogen bromide) coupled column. Peptides not bound to the antibody were removed, and hTregL1-his peptides interacting with GTC310-01-hIgG1 were extracted. The peptides detected by LC-MS, MS$^2$ analysis were identified by amino acid sequencing.

Confirm that hTregL1-his (Ag) binds to the CNBr column conjugated with GTC310-01-hIgG1 (Ab), then load the Ag peptide digested with chymotrypsin or trypsin. Immuno-affinity elution fractions were analyzed by LC-MS/MS to identify Ag-derived peptides. Trypsin analysis results showed that the 499-531 and 630-668 regions were the most highly detected. Chymotrypsin analysis showed that the 402-416 and 430-440 regions were highly detected. As summarized in Table 20, the 402-471 region is a complex disulfide bond-formed area, and there is a possibility that these peptides were trapped and detected regardless of the epitope. Additionally, the regions T103-121, Y113-134, and T630-668, Y637-656, which include N-glycosylation at N116 and N650, were commonly identified; however, the likelihood of these being epitopes is low. Considering the possibility that Ag's epitope is a structural epitope, all test results were comprehensively analyzed to derive the final conclusion.

[Table 20] Affinity-MS: Trypsin

| Trypsin | | | | | | |
|---------|--------|--------|--------|-----------------|----------|------------------|
| Location | MS Area | | | Disulfide bridge | N-glycan | Peptide sequence |
| 103-121 | 273,634 | 389,523 | 984,392 | | 116(N) | AYLSLEVLDLSLNNITEVR (SEQ ID NO: 48) |
| 325-352 | 815,426 | 485,423 | 3,769,1 01 | | | VLDLDHNEISGTIEDTSGAFSGLDSLSK (SEQ ID NO: 49) |
| 498-531 | 7,657,1 99 | 4,643,1 60 | 4,097,1 88 | | | KDNEVLTNADMENFVHVHAQDGEVMEYTTILHLR (SEQ ID NO: 50) |

(continued)

| Trypsin | | | | | | |
|---------|---|---|---|---|---|---|
| Location | MS Area | | | Disulfide bridge | N-glycan | Peptide sequence |
| 499-529 | 1,205,2 21 | 726,243 | 442,272 | | | DNEVLTNADMENFVHVH AQDGEVMEYTTILH (SEQ ID NO: 51) |
| 499-531 | 19,421, 676 | 18,598, 002 | 6,914,5 99 | | | DNEVLTNADMENFVHVH AQDGEVMEYTTILHLR (SEQ ID NO: 52) |
| 630-668 | 10,857, 269 | 9,094,9 70 | 2,269,2 67 | 586-638 | 650(N) | IDDAGVYSCTAQNSAGSI SANATLTVLETPSLVVPL EDR (SEQ ID NO: 53) |
| 669-681 | 784,585 | 296,457 | 1,496,4 69 | 680-729 | | VVSVGETVALQCK (SEQ ID NO: 54) |

[Table 21] Affinity-MS: Chymotrypsin

| Chymotrypsin | | | | | | |
|--------------|---|---|---|---|---|---|
| Location | MS Area | | | Disulfide bridge | N-glycan | Peptide sequence |
| 113-134 | 556,298 | 544,448 | | | 116(N) | SLNNITEVRNTCFPHGP PIKEL (SEQ ID NO: 55) |
| 270-280 | 459,173 | 334,921 | | | | SFCQKLHELVL (SEQ ID NO: 56) |
| 327-344 | 283,198 | 297,866 | | | | DLDHNEISGTIEDTSGA F (SEQ ID NO: 57) |
| 402-416 | 16,681,67 0 | 7,238,269 | | 410-434 / 412-455 | | HISSDSFLCDCQLKW (SEQ ID NO: 58) |
| 427-440 | 1,054,420 | 553,816 | | 410-434 | | QAFVTATCAHPESL (SEQ ID NO: 59) |
| 430-440 | 15,915,26 3 | 11,763,70 2 | | 410-434 | | VTATCAHPESL (SEQ ID NO: 60) |
| 430-446 | 1,004,652 | 705,498 | | 410-434 | | VTATCAHPESLKGQSIF (SEQ ID NO: 61) |

(continued)

| Chymotrypsin | | | | | |
|---|---|---|---|---|---|
| Location | MS Area | | Disulfide bridge | N-glycan | Peptide sequence |
| 447-471 | 1,624,334 | 1,316,956 | 412-455 | | SVPPESFVCDDFLKPQII TQPETTM (SEQ ID NO: 62) |
| 472-492 | 406,738 | 325,171 | 482-543 | | AMVGKDIRFTCSAASS SSSPM (SEQ ID NO: 63) |
| 529-535 | 378,075 | 311,968 | | | HLRQVTF (SEQ ID NO: 64) |
| 536-553 | 497,388 | 295,106 | 482-543 | | GHEGRYQCVITNHFGS TY (SEQ ID NO: 65) |
| 585-598 | 766,101 | 374,036 | 586-638 | | ECAATGHPNPQIAW (SEQ ID NO: 66) |
| 624-636 | 473,456 | 399,084 | | | FITDVKIDDAGVY (SEQ ID NO: 67) |
| 637-653 | 334,084 | 207,267 | 586-638 | 650(N) | SCTAQNSAGSISANATL (SEQ ID NO: 68) |
| 728-744 | 335,107 | 735,656 | 680-729 | | TCEMSNTLGTERAHSQ L (SEQ ID NO: 69) |

### [3-4] Conclusion

To identify the epitope of hTregL1-his, peptide screening, covalent labeling (CL-MS), and cross-linking (XL-MS) assays were performed. The results of all trypsin samples confirmed that the region 477-531 (498-531) was the epitope. Chymotrypsin showed that this region was cleaved into 10 peptides, demonstrating differences in the detected regions depending on the assay method, and was used as supplementary data. The accuracy of the CL-MS and peptide screening results was high, so these two datasets were primarily evaluated.

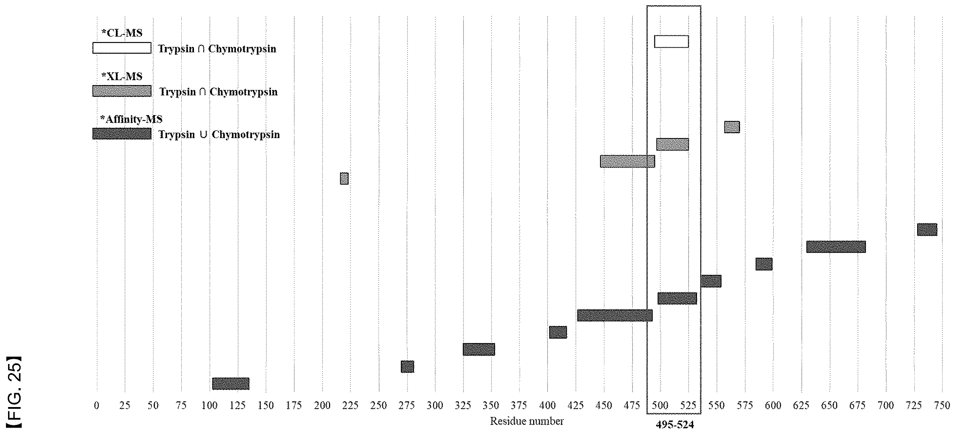

CL-MS (protein surface change analysis) results clearly showed epitopes. The labeled residues were K497 and K556 in the T480-498 (15.42%) and T532-558 (19.02%) regions. The Y495-524 (2.99%) region had a different sequence but the labeled residues K497 and K498 were consistent (Table 14, FIG. 15). Peptide screening (Affinity-MS) results confirmed the region as T499-531 (Tables 20 and 21). The middle sequence regions identified in the Affinity-MS results, rather than the 480-498 and 532-558 regions that appear to be close to the antigen and antibody in CL-MS, are consistent with the Affinity-MS results. These results suggest that the antibody is adjacent to the exposed regions of the antigen, K498 and K556, and recognizes the 499-531 sequence. The absence of the corresponding region in the chymotrypsin analysis of Affinity-MS is likely due to the theoretical generation of 12-14 chymotryptic peptides in the 480-558 (79 amino acid) segment, resulting in the amino acids in the affinity region being cleaved and undetectable. The XL-MS results are opposite to the Affinity-MS analysis, with the undetected region expected to be the epitope, but the T425-497 region was detected at less than 1% in all sequence forms (Tables 20 and 21). Additionally, the T477-531, Y447-494, and Y495-524 regions were detected at levels below 10%, consistent with the results of the other two analyses (FIG. 25).

The sequence segment K497-Y524 (KKDNEVLTNADMENFVHVHAQDGEVMEY: SEQ ID NO: 29) was identified in the molecular structure prediction model (figure omitted). The front part of the b-sheet structure model, corresponding to the CL-MS and PS results, 497-506 (KKDNEVLTNA: SEQ ID NO: 28) and 514-522 (VHAQDGEVM: SEQ ID NO: 30), are exposed externally compared to other parts and are likely to be the regions binding to the antigen. In particular, K497 and K566 are stereochemically close and show a high correlation with the reduced labeling results from CL-MS.

In conclusion, the epitope recognized by GTC310-10-hIgG1 Ab in the hTregL1-his antigen is the sequence 497-506. Additionally, the sequence including 514-522 and the K566 region can be concluded as regions recognized by the antigen or in close proximity to it.

Although the present invention has been described in detail above, the scope of the present invention is not limited thereto. It will be obvious to those skilled in the art that various modifications and changes can be made without departing from the technical spirit of the present invention described in the claims.

## Claims

1. An epitope of Lrig-1 (leucine-rich and immunoglobulin-like domains 1) protein comprising at least one polypeptide selected from the group consisting of polypeptides consisting of the amino acid sequences represented by SEQ ID NOs: 22, 25, 28, 30, 33, 38, 41, 43, 44, 47, 51, and 60.

2. The epitope according to claim 1,
   wherein the polypeptide comprises selected from the group consisting of polypeptides consisting of the amino acid sequences represented by SEQ ID NOs: 21, 23, 24, 26, 27, 31, 32, 34-40, 42, 45, 46, 48-50, 52-59 or 61-69.

3. A nucleic acid molecule encoding the binding molecule of any one of claims 1 to 2.

4. An expression vector into which the nucleic acid molecule of claim 3 is inserted.

5. A host cell line transfected with the expression vector of claim 4.

6. A binding molecule specifically binds to at least one epitope selected from a group consisting of polypeptides consisting of amino acid sequences represented by SEQ ID NOs: 21-69.

7. The binding molecule according to claim 6,
   wherein the binding molecule is an antibody or an antigen-binding fragment thereof.

8. The binding molecule according to claim 7,
   wherein the antibody is a chimeric antibody, a humanized antibody, a bivalent, bispecific molecule, a minibody, a domain antibody, a bispecific antibody, an antibody mimetic, a unibody, a diabody, a triabody, or a tetrabody, or a fragment thereof.

9. A chimeric antigen receptor (CAR) comprising an antigen-specific binding domain, a linking domain and a CD3 zeta (ζ) signaling domain,
   wherein the antigen-specific binding domain is a fusion protein comprising an antigen-specific binding domain specifically binds to at least one epitope selected from a group consisting of polypeptides consisting of amino acid sequences represented by SEQ ID NOs: 21-69; and an immunoglobulin Fc region.

10. An antibody-drug conjugate comprising the binding molecule claim 6; and a drug.

11. A pharmaceutical composition for the prevention or treatment of a brain nervous system disease, comprising as an effective ingredient one or more of the binding molecules of any one of claims 6 to 8, the chimeric antigen receptor of claim 9, and the antibody-drug conjugate of claim 10.

12. The pharmaceutical composition according to claim 11,
    wherein the brain and nervous system disease is a neurodegenerative or neuroinflammatory disease.

13. The pharmaceutical composition according to claim 12,
    wherein the neurodegenerative or neuroinflammatory disease is selected from the group consisting of stroke, dementia, Alzheimer's disease, Parkinson's disease, Huntington's disease, Niemann-Pick disease, multiple sclerosis, prion disease, Creutzfeldt-Jakob disease, frontotemporal dementia, dementia with Lewy bodies, amyotrophic lateral sclerosis, paraneoplastic syndrome, cortical degeneration syndrome, multiple system atrophy, progressive supranuclear palsy, nervous system autoimmune disease, spinocerebellar ataxia, inflammatory and neuropathic pain, cerebrovascular disease, spinal cord injury, and tauopathy.

14. A method for screening a binding molecule, such as antibodies or their fragments, that specifically bind to the epitope of claim 1 or 2.

15. A method for screening the epitope of Lrig-1 protein using a binding molecule of any one of claims 6 to 8.

16. A method for binding the binding molecule, which is an antibody or an antigen-binding fragment thereof, to the epitope of any one of claim 1 or 2.

17. An antibody or antigen-binding fragment that binds specifically to Lrig-1 (leucine-rich and immunoglobulin-like domains 1) protein comprising:

   (a) a heavy chain variable region comprising a heavy chain CDR1 consisting of the amino acid sequence represented by SEQ ID NO: 7, a heavy chain CDR2 consisting of the amino acid sequence represented by SEQ ID NO: 8, and a heavy chain CDR3 consisting of the amino acid sequence represented by SEQ ID NO: 9; and
   (b) a light chain variable region comprising a light chain CDR1 consisting of the amino acid sequence represented by SEQ ID NO: 10, a light chain CDR2 consisting of the amino acid sequence represented by SEQ ID NO: 11, and a light chain CDR3 consisting of the amino acid sequence represented by SEQ ID NO: 12,

   wherein the binding molecule specifically binds to at least one epitope selected from a group consisting of polypeptides consisting of amino acid sequences represented by SEQ ID NOs: 21-69.

18. The binding molecule according to claim 17,
   wherein the binding molecule comprises:

   a heavy chain variable region consisting of the amino acid sequence represented by SEQ ID NO: 13; or
   a light chain variable region consisting of the amino acid sequence represented by SEQ ID NO: 14.

19. The binding molecule according to claim 18,
   wherein the binding molecule comprises:

   a heavy chain consisting of the amino acid sequence represented by SEQ ID NO: 15 or 17; or
   a light chain consisting of the amino acid sequence represented by SEQ ID NO: 16 or 18.

20. A method for preventing or treating a brain nervous system disease, comprising administering to an individual an effective amount of any one or more of the following: a binding molecule according to any one of claims 6 to 8; a chimeric antigen receptor according to claim 9; an antibody-drug conjugate according to claim 10; or a binding molecule according to any one of claims 17 to 19.

21. The method according to claim 20,
   wherein the brain and nervous system disease is a neurodegenerative or neuroinflammatory disease.

22. The method according to claim 21,
   wherein the neurodegenerative or neuroinflammatory disease is selected from the group consisting of stroke, dementia, Alzheimer's disease, Parkinson's disease, Huntington's disease, Niemann-Pick disease, multiple sclerosis, prion disease, Creutzfeldt-Jakob disease, frontotemporal dementia, dementia with Lewy bodies, amyotrophic lateral sclerosis, paraneoplastic syndrome, cortical degeneration syndrome, multiple system atrophy, progressive supranuclear palsy, nervous system autoimmune disease, spinocerebellar ataxia, inflammatory and neuropathic pain, cerebrovascular disease, spinal cord injury, and tauopathy.

【FIG. 1】

| | Mice strain | Mice No. | Test article | Dosing regimen | | | Note |
|---|---|---|---|---|---|---|---|
| | | | | Dose | Frequency | Route | |
| G1 | WT | 12 | Vehicle | - | - | | WT Ctrl |
| G2 | | 10 | Vehicle | - | - | | Tg Ctrl |
| G3 | 5xFAD | 8 | Glatiramer acetate | 100 ug | BIW+QW x 3 wks | SC | Immune modulator (random polymer composed of four AA of MBP) |
| G4 | | 10 | GTC110-04 | 10 mpk | BIW x 4 wks | IV | anti-Lrig1 antibody |
| G5 | | 10 | GTC310-01 | 10 mpk | BIW x 3 wks | IV | anti-Lrig1 antibody |

【FIG. 2】

5xFAD Tg    GA    GTC110-04    GTC310-01

【FIG. 3】

【FIG. 4】

A: WT
B: 5xFAD Tg
C: +GA
D: GTC110-04
E: GTC310-01

【FIG. 5】

A: WT
B: 5xFAD Tg
C: +GA
D: GTC110-04
E: GTC310-01

【FIG. 6】

A: WT
B: 5xFAD Tg
C: +GA
D: GTC110-04
E: GTC310-01

【FIG. 7】

A: WT
B: 5xFAD Tg
C: +GA
D: GTC110-04
E: GTC310-01

【FIG. 8】

MWM-Acquisition test

— WT
— 5xFAD Tg
-- +GA
— GTC110-04
--*-- GTC310-01

**【FIG. 9】**

| | Group | Number |
|---|---|---|
| WT | Vehicle | 7 |
| 5xFAD(Female) | Vehicle | 6 |
| | GTC310-01 | 6 |
| 6xTg(Female) | Vehicle | 7 |
| | GTC310-01 | 8 |

**【FIG. 10】**

Y-maze test

$$Spontaneous\ alternation(\%) = \frac{(Number\ of\ alternation)}{(Total\ arm\ entries-2)} * 100$$

【FIG. 11】

【FIG. 12】

1ˢᵗ day(Adaptation)    2ⁿᵈ day(Acquisition Trial)    3ʳᵈ day(Retention Trial)

PAT in 5xFAD

PAT in 6xTg

【FIG. 13】

[FIG. 14]

【FIG. 15】

【FIG. 16】

【FIG. 17】

【FIG. 18】

【FIG. 19】

【FIG. 20】

【FIG. 21】

【FIG. 22】

【FIG. 23】

【FIG. 24】

【FIG. 25】

## INTERNATIONAL SEARCH REPORT

| International application No. |
|---|
| **PCT/KR2024/003075** |

| A. | CLASSIFICATION OF SUBJECT MATTER |
|---|---|

**C07K 14/47**(2006.01)i; **C07K 16/18**(2006.01)i; **C07K 14/725**(2006.01)i; **A61K 47/68**(2017.01)i; **A61P 37/00**(2006.01)i; **A61P 25/28**(2006.01)i; **G01N 33/68**(2006.01)i; **A61K 39/00**(2006.01)i

According to International Patent Classification (IPC) or to both national classification and IPC

| B. | FIELDS SEARCHED |
|---|---|

Minimum documentation searched (classification system followed by classification symbols)

C07K 14/47(2006.01); A61K 39/00(2006.01); A61K 47/68(2017.01); C07K 14/705(2006.01); C07K 16/18(2006.01); C12Q 1/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 조절 T 세포(regulatory T cell, Treg cell), Lrig-1(leucine-rich and immunoglobulin-like domains 1), 에피토프(epitope), 항체(antibody)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2021-0056280 A (GOOD T CELLS, INC.) 18 May 2021 (2021-05-18) See abstract; SEQ ID NO: 66; paragraph [0055]; and claims 1, 3, 5-7 and 10. | 1-8,10 |
| Y | | 9,11-19 |
| Y | KR 10-2023-0016148 A (GOOD T CELLS, INC.) 01 February 2023 (2023-02-01) See paragraph [0175]; and claims 9 and 14-20. | 9,11-19 |
| Y | KR 10-2022-0108747 A (GOOD T CELLS, INC.) 03 August 2022 (2022-08-03) See abstract; and claims 1 and 22-25. | 9,11-13 |
| A | WO 2020-070290 A1 (ST. ANNA KINDERKREBSFORSCHUNG et al.) 09 April 2020 (2020-04-09) See entire document. | 1-19 |

| ☑ Further documents are listed in the continuation of Box C. | ☑ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **24 June 2024** | **27 June 2024** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2024/003075**

### C.    DOCUMENTS CONSIDERED TO BE RELEVANT

| Category* | Citation of document, with indication, where appropriate, of the relevant passages | Relevant to claim No. |
|---|---|---|
| A | KR 10-1847523 B1 (INDUSTRY-ACADEMIC COOPERATION FOUNDATION, YONSEI UNIVERSITY) 28 May 2018 (2018-05-28)<br>See entire document. | 1-19 |

Form PCT/ISA/210 (second sheet) (July 2022)

**INTERNATIONAL SEARCH REPORT**

International application No.

**PCT/KR2024/003075**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/KR2024/003075** |

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **20-22**
   because they relate to subject matter not required to be searched by this Authority, namely:

   Claims 20-22 pertain to a method for treatment of the human body, and thus pertain to a subject matter on which the International Searching Authority is not required to carry out an international search under the provisions of PCT Article 17(2)(a)(i) and PCT Rule 39.1(iv).

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/KR2024/003075**

| Patent document cited in search report | | | Publication date (day/month/year) | Patent family member(s) | | | Publication date (day/month/year) |
|---|---|---|---|---|---|---|---|
| KR | 10-2021-0056280 | A | 18 May 2021 | AU | 2020-380072 | A1 | 19 May 2022 |
| | | | | BR | 112022008730 | A2 | 19 July 2022 |
| | | | | CA | 3156600 | A1 | 14 May 2021 |
| | | | | CN | 114929733 | A | 19 August 2022 |
| | | | | EP | 4056586 | A1 | 14 September 2022 |
| | | | | JP | 2023-500248 | A | 05 January 2023 |
| | | | | KR | 10-2488967 | B1 | 16 January 2023 |
| | | | | WO | 2021-091359 | A1 | 14 May 2021 |
| KR | 10-2023-0016148 | A | 01 February 2023 | CN | 117716238 | A | 15 March 2024 |
| | | | | EP | 4375670 | A1 | 29 May 2024 |
| | | | | WO | 2023-287212 | A1 | 19 January 2023 |
| KR | 10-2022-0108747 | A | 03 August 2022 | AU | 2022-211952 | A1 | 17 August 2023 |
| | | | | BR | 112023015002 | A2 | 05 December 2023 |
| | | | | CA | 3206270 | A1 | 04 August 2022 |
| | | | | CN | 116829589 | A | 29 September 2023 |
| | | | | EP | 4286412 | A1 | 06 December 2023 |
| | | | | JP | 2024-505210 | A | 05 February 2024 |
| | | | | WO | 2022-164231 | A1 | 04 August 2022 |
| WO | 2020-070290 | A1 | 09 April 2020 | CA | 3109747 | A1 | 09 April 2020 |
| | | | | CN | 113164576 | A | 23 July 2021 |
| | | | | EP | 3632461 | A1 | 08 April 2020 |
| | | | | EP | 3860643 | A1 | 11 August 2021 |
| | | | | JP | 2022-512594 | A | 07 February 2022 |
| | | | | KR | 10-2021-0075117 | A | 22 June 2021 |
| | | | | US | 2021-0386781 | A1 | 16 December 2021 |
| KR | 10-1847523 | B1 | 28 May 2018 | KR | 10-1959237 | B1 | 20 March 2019 |
| | | | | KR | 10-2020-0035924 | A | 06 April 2020 |
| | | | | KR | 10-2067402 | B1 | 17 January 2020 |
| | | | | KR | 10-2094747 | B1 | 30 March 2020 |
| | | | | KR | 10-2317415 | B1 | 28 October 2021 |
| | | | | US | 11091538 | B2 | 17 August 2021 |
| | | | | US | 11655287 | B2 | 23 May 2023 |
| | | | | US | 11932686 | B2 | 19 March 2024 |
| | | | | US | 2015-0239964 | A1 | 27 August 2015 |
| | | | | US | 2019-0185552 | A1 | 20 June 2019 |
| | | | | US | 2020-0048335 | A1 | 13 February 2020 |
| | | | | US | 2021-0340235 | A1 | 04 November 2021 |
| | | | | US | 2022-0135662 | A1 | 05 May 2022 |
| | | | | WO | 2012-102527 | A2 | 02 August 2012 |
| | | | | WO | 2012-102527 | A3 | 20 December 2012 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 4554101 A